# EUROPEAN PATENT APPLICATION

(11) **EP 3 730 607 A1**
(43) Date of publication of application: **28.10.2020**
(21) Application number: 20177392.6
(22) Date of filing: 31.03.2017
(51) Int. Cl.: C12N 5/0775, C12N 5/0783, A61K 35/28, A61K 35/17, C07K 16/30, C12N 5/077, C07K 14/54, A61K 39/00, A61P 35/00, C07K 16/28

(54) **MESENCHYMAL STEM CELLS TO ENHANCE ANTI-TUMOR ACTIVITY OF IMMUNOTHERAPY**

(30) Priority: 01.04.2016 EP 16163557; 24.06.2016 EP 16176185
(62) Divisional of application: 17715658.5
(71) Applicant: Apceth GmbH & Co. KG, 81377 München (DE)
(72) Inventor: Hermann, Felix, 81375 München (DE); Günther, Christine, 81479 München (DE); Geumann, Ulf, 81677 München (DE)
(74) Representative: Hertin und Partner Rechts- und Patentanwälte PartG mbB

(57) **Abstract**

The invention relates to mesenchymal stem cells (MSCs) for use in the treatment of a tumor, wherein said treatment comprises the combined administration of said mesenchymal stem cells with an anti-tumor immunotherapy, and wherein said MSCs do not comprise exogenous nucleic acids that encode immune response-stimulating cytokines. In a preferred embodiment the invention relates to the use of said MSCs in the treatment of a tumor and/or malignant disease, wherein the anti-tumor immunotherapy for combined administration comprises the administration of an immune checkpoint modulator, preferably of an immune checkpoint inhibitor, and the mesenchymal stem cells are not genetically modified.

## Description

The invention relates to mesenchymal stem cells (MSCs) for use in the treatment of a tumor, wherein said treatment comprises the combined administration of said mesenchymal stem cells with an anti-tumor immunotherapy, and wherein said MSCs do not comprise exogenous nucleic acids that encode immune response-stimulating cytokines. In embodiments of the invention the MSCs are either not genetically modified, or are genetically modified but comprise exogenous nucleic acids that do not encode immune-stimulatory cytokines and/or do not encode other immune-stimulatory molecules that induce an antigen-independent immune response and/or stimulation.

In a preferred embodiment the invention relates to the use of said MSCs in the treatment of a tumor and/or malignant disease, wherein the anti-tumor immunotherapy for combined administration comprises the administration of a cellular immunotherapy, preferably chimeric antigen receptor (CAR) T cells, wherein said T cell receptor binds specifically to a tumor-associated antigen, and the mesenchymal stem cells are not genetically modified.

The invention encompasses the use of MSCs in the treatment of a tumor and/or malignant disease, for example by modulating the tumor microenvironment in order to attract and/or stimulate immune cells that exhibit an anti-tumor effect. One aspect of the invention relates to the use of said MSCs in anti-tumor treatment comprising the combined administration of said mesenchymal stem cells with immune cells, for example T cells, such as T cells with artificial T cell receptors, for example a chimeric antigen receptor (CAR-Ts), or T-Cell Receptor (TCR) transduced cells, NK cells or macrophages/monocytes. Furthermore, additional immunotherapy approaches, for example checkpoint inhibitors, including for example antibodies against CTLA-4, PD-1, PD-L1 or other immune co-stimulatory molecules, or active immunotherapeutic drugs, for example, tumor antigens, patient-derived tumor material and other therapeutic drugs aiming to activate and/or direct the immune response against a tumor, or features of a tumor, may be combined with the administration of the MSC.

### BACKGROUND OF THE INVENTION

Mesenchymal stem cells (MSCs) are cells of non-haematopoietic origin that reside in the bone marrow and other tissues. The correct identification and nomenclature of the MSC has been clarified by the Mesenchymal and Tissue Stem Cell Committee of the International Society for Cellular Therapy (ISCT), who have proposed a set of minimal criteria to define MSC: Firstly, MSC grow in a plastic-adherent manner when maintained under standard cell culture conditions. Secondly, the cells express a panel of the following surface markers: CD105, CD73, and CD90 expression (≥95%). On the other hand, a set of specific markers must be absent: CD45, CD34, CD14, or CD11b, CD79alpha or CD19 and HLA-DR (≤2% positive). Furthermore, the MSC must be able to differentiate into osteoblasts, adipocytes, and chondroblasts when cultured under standard in vitro differentiating conditions (M. Dominici, et al., 2006, Cytotherapy, vol. 8, no. 4, pp. 315-317).

The isolation, cultivation and administration of MSCs has been identified as promising approach towards treating inflammation-associated disorders. This conclusion was based on the initial observation that MSCs are able to inhibit mixed lymphocyte reactions (MLR) in vitro. It was shown that native MSCs are able to suppress T-lymphocyte proliferation in both MLR and in the presence of polyclonal-activators (interleukin (IL)-2 or Phytohemagglutinin (PHA)) (M. D. Nicola, et al., 2002, Blood, vol.99, no. 10, pp. 3838-3843,).

The suppression of T cell activation by MSCs can be shown in cultures containing peripheral blood mono nuclear cells or purified T cells. (Wang et al, 2014, Arthritis Rheumatol. Aug 66(8): 2234-45). The suppression of T cell responses by MSCs has been widely studied and has been confirmed by many independent researchers, such as Bartholomew et al (Mesenchymal stem cells suppress lymphocyte proliferation in vitro and prolong skin graft survival in vivo. Exp Hematol. 2002, 30: 42-48), Aggarwal and Pittenger (Human mesenchymal stem cells modulate allogeneic immune cell responses. Blood. 2005, 105: 1815-1822) and Krampera et al (Bone marrow mesenchymal stem cells inhibit the response of naive and memory antigen-specific T cells to their cognate peptide. Blood. 2003, 101: 3722-3729.).

Because of the ability of MSC to suppress T cell activation, MSCs have been proposed to be beneficial to treat inflammatory diseases. MSCs have therefore been extensively studied in clinical trial with the aim to treat graft-versus host disease (GvHD). GvHD is a potential side effect of hematopoietic stem cell transplantation, in which donor derived-T cells may attack the host. In relation to GvHD, MSC have been tested in clinical trials with the aim to suppress the allo-reactive donor T cells and thereby to alleviate GvHD symptoms (Resnick et al. Treatment of severe steroid resistant acute GVHD with mesenchymal stromal cells (MSC); American Journal of Blood Research, 2013;3(3):225-238., and Herrmann et al, Adult human mesenchymal stromal cells and the treatment of graft versus host disease, Stem Cells and Cloning: Advances and Applications, 2014;7:45-52.).

MSCs are known to exhibit immune evasive properties after administration to a patient. MSCs have been shown to exhibit a immunomodulatory / T cell suppressive effect in cases of transplantation of allogeneic donor material (Le Blanc et al, Lancet 2004: 363, p 1439), thereby reducing a potentially pathogenic alloreactivity and rejection. MSCs treatment can also play a therapeutic role in wound healing. The therapeutic delivery of MSCs can be performed via systemic injection, followed by MSC homing to and engraftment within sites of injury (Kidd et al, Stem Cells 2009: 27, p 2614). MSCs have also been described as vehicles for directing anti-cancer agents to tumors, for example in WO 2008/150368, which describes the transgenic expression of HSV-TK from MSCs at tumor sites and the combined systemic administration of ganciclovir.

Artificial T cell receptors (also known as chimeric T cell receptors, chimeric immunoreceptors, chimeric antigen receptors (CARs)) are engineered receptors, which graft an arbitrary specificity onto an immune cell. Typically, these receptors are used to graft the specificity of a monoclonal antibody onto a T cell and to activate that T cell upon cognate antigen engagement; with transfer of their coding sequence facilitated by retro- or lentiviral vectors. The receptors are called chimeric because they are composed of parts from different sources.

Artificial T cell receptors are under investigation as a therapy for cancer, using a technique called adoptive cell transfer. T cells are removed from a patient and modified ex vivo so that they express receptors specific to the particular form of cancer. The T cells, which can then recognize and kill the cancer cells, are administered into the patient with or without pre-conditioning of the patient. Allogeneic T cells engineered in the same way and sourced from donors other than the patient are also under investigation.

Despite progress in inducing anti-tumor immune response using immunotherapies, such as CAR-T cells, difficulties remain in achieving sufficient induction of the desired immune response in order to show the desired therapeutic effect.

The prior art discloses means for enhancing anti-tumor immune responses. WO 2016/026854 discloses genetically modified MSCs expressing immune-response stimulating cytokines to attract and/or activate immune cells in response against a tumor. According to WO 2016/026854, the expression of an immune-stimulating cytokine from an exogenous nucleic acid is necessary for activation of the desired immune response. The administration of MSCs, either not genetically modified or without exogenous nucleic acids that encode immune-stimulatory cytokines, is neither disclosed nor suggested.

WO 2014/087217 discloses the administration of a mixture of allogenic dendritic cells and MSCs isolated from donated cord blood and primed with antigens from autologous tumor cells for the treatment of tumors. WO 2014/087217 teaches the use of dendritic cells in presenting tumor antigens and stimulating endogenous T cells for the desired immune response. No mention is made of MSCs stimulating an anti-tumor immune response.

Hass et al (Cell Communication and Signaling, 2012, v. 10, no. 1, 26) presents a summary of MSC functions in neoplastic microenvironments and discloses the immune suppressive capacity of MSCs, in particular the suppression of T cells, dendritic cells and Natural Killer cells by MSCs. Zhang et al (Stem Cell Research and Therapy, 2015, v. 6, no. 1, 45) also discloses the suppression of the function of a variety of immune cells, including T and B lymphocytes, by MSCs. Prigione et al (Stem Cells, 2009, v. 27, no. 3, 693) discuss the MSC-mediated impairment of T cell proliferation for particular T cell subsets that are relevant in the treatment of autoimmune diseases.

Ningxia Song et al (Int. J. Mol. Medicine, 2015, 36: 139-149) disclose that MSCs, when administered in combination with A20 lymphoma cells, lead to improved survival in mouse models, albeit dependent on a direct anti-proliferative effect on lymphoma cells and cell-cell contacts. Fritz et al (Current Stem Cell Research and Therapy, 2008, v. 3, no. 1, 32-34) present a review of MSCs and their uses in anti-tumor therapy, discussing the immunosuppressive effects of MSCs and their use as delivery vehicles for anti-cancer agents. Loebinger et al (Cancer Research, 2009, v. 69, no. 10, 4134) describe genetically engineered MSCs expressing an apoptosis-inducing ligand TRAIL from an exogenous transgene and their use in the treatment of cancer. Neither the combined administration of MSCs with anti-tumor immune therapies nor MSC-enhancement of an anti-tumor immune response is disclosed in these documents.

Although adoptive cell therapy is effective in treating some cancers, including melanoma and leukemia/lymphoma, the therapy still fails in the treatment of most other malignancies, in particular in most cases of solid cancer. There remains a major medical need to improve the anti-cancer activity of immunotherapies, in particular of cellular immunotherapies such as those based on T cell administration, for example for both genetically modified (CAR-Ts) and unmodified immune cells.

### SUMMARY OF THE INVENTION

In light of the prior art the technical problem underlying the present invention is to provide improved or alternative means for immunotherapy of cancers, or to provide means for improving existing immunotherapies for the treatment of cancer. In particular, a problem underlying the invention is the provision of suitable means for stimulation, such as local stimulation, of an immune response directed against cancerous cells and/or tumor tissue in the patient. A further problem underlying the invention is the provision of means for enhancing immune cell, in particular CAR-T, activity against cancerous cells or tissue.

This problem is solved by the features of the independent claims. Preferred embodiments of the present invention are provided by the dependent claims.

The invention therefore relates to mesenchymal stem cells (MSCs) for use in the treatment of a tumor, wherein said treatment comprises the combined administration of said mesenchymal stem cells with an anti-tumor immunotherapy and wherein said MSCs do not comprise exogenous nucleic acids that encode immune response-stimulating cytokines.

The invention therefore relates in one embodiment to mesenchymal stem cells (MSCs) for use in the treatment of a tumor, wherein said treatment comprises the combined administration of said mesenchymal stem cells with an anti-tumor immunotherapy and wherein said MSCs are not genetically modified.

The invention therefore relates in one embodiment to mesenchymal stem cells (MSCs) for use in the treatment of a tumor, wherein said treatment comprises the combined administration of said mesenchymal stem cells with an anti-tumor immunotherapy and wherein said MSCs are genetically modified, but do not comprise exogenous nucleic acids that encode immune response-stimulating cytokines.

The invention therefore relates in one embodiment to mesenchymal stem cells (MSCs) for use in the treatment of a tumor, wherein said treatment comprises the combined administration of said mesenchymal stem cells with an anti-tumor immunotherapy and wherein said MSCs are genetically modified, but do not comprise exogenous nucleic acids that encode immune-stimulatory molecules that induce an antigen-independent immune response.

The invention therefore encompasses the medical use of the MSCs described herein, in particular for treating cancer, in addition to methods for the treatment of a subject with a tumor, or methods of treatment of a tumor and/or tumor disease.

The present invention is based on the surprising finding that human MSCs that are not genetically modified, in particular those MSC without transgenes encoding immune-stimulatory cytokines or other transgenic products that show antigen-independent immune stimulatory properties, are able to enhance the activity of an anti-tumor immunotherapy. This finding is particularly unexpected, as MSCs have been previously described, and are commonly accepted, to exhibit immunosuppressive functions. As mentioned in detail above, the prior art discloses that MSCs may be employed in the treatment of autoimmune diseases and GVHD, namely in diseases in which immune suppression is the desired therapeutic outcome.

In particular, it was surprising that genetically unmodified MSCs enhance the anti-tumor activity of such a combined immunotherapy to a similar (but somewhat reduced) extent as genetically modified MSCs comprising exogenous nucleic acid molecules encoding antigen-unspecific immune-stimulatory molecules, in particular immune-stimulatory cytokines, which are secreted from these cells.

The present invention is preferably directed to MSCs that are not genetically modified. In other embodiments the MSCs may exhibit a genetic modification, but do not exhibit a transgene encoding one or more immune stimulatory molecules such as immune stimulatory cytokines, which are considered to induce an antigen-independent immune response. The invention is directed towards the finding that although MSCs are thought to suppress immune responses in vivo, the MSC used in the examples below show properties of stimulating an anti-tumor immune response from a CAR-T cell.

Preferred embodiments of the invention also includes MSCs which are genetically modified to furthermore enhance the supportive effect on the anti-tumor response, for example through the expression of cytotoxic transgenes. In a preferred embodiment such transgenes, for example cytotoxic transgenes, do not lead to an antigen-unspecific immune stimulation.

The MSCs as described herein preferably migrate to tumor tissue due to either their natural or an engineered capacity for homing to areas of inflammation in vivo. The homing to and/or engraftment into tumor tissue (tumor stroma) may lead to local expression of immune stimulating molecules such as cytokines from endogenous nucleic acids, thereby enabling higher anti-tumor efficacy of endogenous or administered immune cells at the site of a tumor.

The invention provides suitable means for local stimulation of an immune response directed against tumor tissue in a subject. This includes in particular immunotherapeutic treatments aiming to direct the immune response against the tumor. Such support or induction of the immune response may in various clinical settings be beneficial in order to initiate and maintain the immune response and evade the tumor-mediated immunosuppression that often blocks this activation.

The mesenchymal stem cells of the present invention may be used to enhance the activities and/or increase amounts of endogenous immune cells that are already present in the subject. Alternatively or additionally, additional immune cells (either autologous or allogeneic) may be administered in combination with the MSCs (for example concurrently or sequentially) of the invention in order to enhance the desired therapeutic immune response.

In one embodiment the invention therefore relates to cancer immunotherapy and approaches involving adoptive cell transfer, which encompasses T cell-based cytotoxic responses to attack cancer cells.

In one embodiment the invention therefore relates to cancer immunotherapy and approaches involving adoptive cell transfer, which encompasses NK cell-based cytotoxic responses to attack cancer cells.

The present invention encompasses the use of the MSCs as a medicament and/or the use of the MSCs as an adjuvant for anti-tumor immune therapies, preferably those described herein. An adjuvant relates typically to a pharmacological or immunological agent that modifies the effect of other agents. In the present invention the adjuvant (MSCs) leads preferably to an enhanced effect of the anti-tumor immune therapy, that is measureable compared to when no MSC adjuvant is applied. Suitable assays are described in the examples below.

In a preferred embodiment of the present invention, the anti-tumor immunotherapy for combined administration comprises the administration of immune cells. Such therapies involve the administration of immune cells to a tumor patients resulting in an enhanced immune response against the tumor, either due to the direct anti-tumor activity of the administered cells or due to their immune-modulatory activity leading to the activation of endogenous immune cells. According to the present invention, co-administration of genetically unmodified or modified MSCs increases these anti-tumor activity of the transferred immune cells.

A crucial limitation in the successful development and clinical use of immunotherapies, in particular cell therapies, is the ability of tumors to evade and suppress an immune response against the tumor cells by establishing an immunosuppressive tumor microenvironment. This phenomenon is known as tumor-mediated immunosuppression and is mediated to a large extent by the secretion of anti-inflammatory cytokines by immune cells present in the tumor that display a regulatory phenotype (for example, Regulatory T-cells, Tregs; and Monocyte-Derived Suppressor Cells, MDSCs; and tumor associated macrophages, TAMs). The invention therefore provides means to modify the tumor microenvironment, using MSCs as described herein to provide an activating impulse to immune cells, in particular CAR-T cells directed against tumor tissue.

In another preferred embodiment of the present invention, the immune cells for combined administration are T cells. T cells play a central role in cell-mediated immunity and can have cytotoxic as well as immune-modulating functions and through the formation of memory T cells can grant long term protection. T cells play a critical role in anti-tumor immune responses. According to the present invention combined administration of MSCs and T cells leads to an enhanced anti-tumor activity of administered T cells.

In other embodiments, the immune cell is a T cell comprising an artificial T cell receptor, such as a chimeric antigen receptor (CAR-Ts), wherein said T cell receptor binds specifically to a tumor antigen (Lee, DW et al., Clin Cancer Res; 2012;18(10); 2780-90). In a further embodiment the T cells is an ex vivo expanded T cell or a TCR-transgenic T cell.

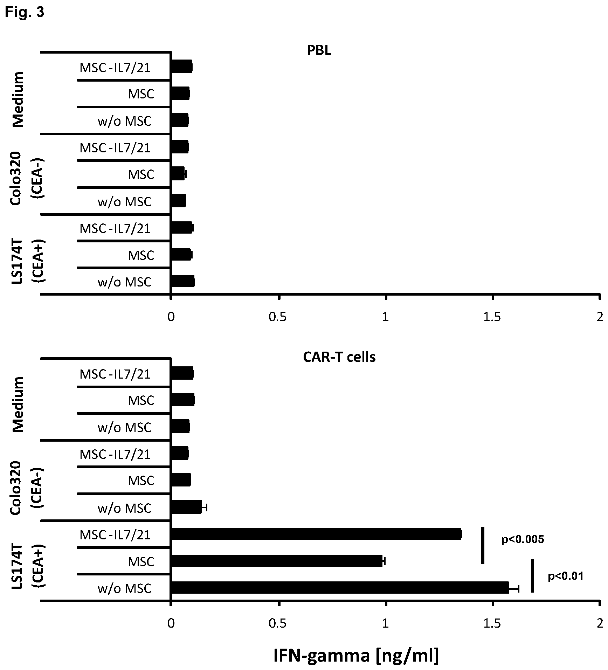

In another preferred embodiment of the present invention, the immune cells for combined administration are chimeric antigen receptor (CAR-Ts) T cells, wherein said T cell receptor binds specifically to a tumor-associated antigen. The present invention is based on the surprising finding that human MSCs are able to enhance the activity of CAR-T cells (Fig. 3-5), despite the fact that MSCs are considered to suppress proliferation of native T cells. In contrast to what would be expected from the literature, human MSCs support CAR-T proliferation in culture, enhance their survival and increase their anti-tumor activity. The invention relates to the application of human MSCs to enhance anti-tumor activity of CAR-T cells with the aim to improve the treatment of solid tumors.

It was entirely surprising that the combined administration of such CAR-T cells together with genetically unmodified MSCs showed an improved therapeutic effect over the treatment with CAR-T cells alone. This shows that the present invention enables the stimulation of CAR-Ts involved in an anti-tumor immune response and thereby the local activation, support and/or strengthening of an anti-tumor immune response. Furthermore, the unique properties of MSCs lead to a maintained enhancement of the CAR-T immune response for an improved and prolonged therapeutic effect.

In one embodiment the MSCs as described herein can be administered prior to, after, or at the same time as CAR-T cell treatment in order to modify and render the tumor microenvironment favorable to CAR-T treatment. Single or multiple transfers of MSCs are possible.

In another embodiment of the present invention, the immune cells for combined administration are dendritic cells. Dendritic cells are the professional antigen-presenting cells of the innate immune system with the potential to generate robust antigen-specific T cell immune responses. Immunotherapeutic strategies have attempted to monopolize on this ability of dendritic cells to deliver antigens as a means of therapeutic vaccination in individuals with advanced malignancies, specifically cancer. Combined administration of therapeutic dendritic cells together with MSCs will optimize dendritic cell vaccines in order to improve antitumor responses in cancer patients.

In another embodiment of the invention the immune cells for combined administration are not dendritic cells, and/or do not comprise dendritic cells.

Another preferred embodiment of the present invention relates the administration of macrophages and/or monocytes as an anti-tumor immunotherapy in combination with mesenchymal stem cell. Monocytes and macrophages are often found in close proximity or within tumor masses and form the major leukocytic infiltrate within the stroma of many tumor types. They are involved in both pro-tumor (e.g. promotion of growth and metastasis through tumor angiogenesis) as well as anti-tumor (tumoricidal and tumorostatic) processes and critically shape the immune status of the tumor microenvironment. Adoptive transfer of monocyte and/or macrophages, which can be pretreated by external stimulation or genetic modification to induce a desired anti-tumor activity, has been suggested as anti-tumor immunotherapy. In a preferred embodiment the administered macrophages are M1 macrophages. The present invention relates to combined administration of such immune cells together with MSCs to further enhance the anti-tumor immune response.

In another preferred embodiment of the present invention, the immune cells for combined administration are innate lymphoid cells, preferably NK cells. Innate lymphoid cells are a group of innate immune cells that belong to the lymphoid lineage but do not respond in an antigen-specific manner and include also the cytotoxic NK cells. NK cells play an important role in tumor surveillance and can be used for treating tumor patients by means of adoptively transfer. It is envisioned that combined administration of MSCs and innate lymphoid cells, preferably NK cells, leads to an enhanced anti-tumor acitivity as compared to treatment with innate lymphoid cells alone. Innate lymphoid cells may be genetically modified or unmodified and may undergo external stimulation before transfer to induce a favorable phenotype.

In another preferred embodiment of the present invention, the immune cells for combined administration are granulocytes. Granulocytes, which include neutrophils, eosinophils, basophils and mast cells, are known to be involved in the orchestration of an anti-tumor immune response. Adoptive transfer of granulocytes, especially eosinophils, has been shown to enhance the immune response against tumors by enhancing cytotoxic T cell activity against tumor cells. The present invention therefore relates to the combined administration of MSCs and granulocytes leading to an enhanced anti-tumor activity of the granulocytes. Transferred granulocytes may be genetically modified or unmodified and may undergo external stimulation before transfer to induce a favorable phenotype.

In one embodiment of the present invention the mesenchymal stem cell and/or the immune cells for combined administration are autologous to the subject of medical treatment. Administration of autologous cells is advantageous because rejection of the transferred cells is very rare due to the donor and recipient being the same individual and because graft versus host disease cannot develop,

In another embodiment of the present invention the mesenchymal stem cell and/or the immune cells for combined administration are allogenic to the subject of medical treatment. Allogeneic transplantation has been established for cellular therapy and enables pre-prepared or commercial preparations of therapeutic cells to be elected, sourced and administered without having to obtain, culture and expand cells prior to administration to the patient. The risk of rejection of the administered allogeneic material can be assessed using common techniques, for example by assessing HLA compatibility between donor and recipient.

In another preferred embodiment of the present invention the anti-tumor immunotherapy for combined administration comprises the treatment with an antibody, cytokine, chemokine or other biopharmaceutical. A successful immunotherapy of an established tumor is typically based on not only the generation of systemic immunity but also overcoming of immune suppression at the tumor site. For example, blockade of the B7-H1/PD-1 pathway by monoclonal antibodies selectively and effectively modulates immune responses at the tumor site. This specificity is largely due to the selective induction and expression of B7-H1 in the tumor microenvironment. This selectivity not only allows a more "targeted" augmentation of the immune response but also avoids the consequences of a broad, systemic autoimmune toxicity. This kind of treatment with a biopharmaceutical drug can be combined with the administration of MSCs, which migrate to the tumor and induce changes in the tumor microenvironment, supporting the induction of a potent anti-tumor immune response by endogenous immune cells and inducing a synergistic effect.

In another preferred embodiment of the present invention the anti-tumor immunotherapy for combined administration comprises the treatment with a small molecule, which has immunomodulatory functions. Modulating the immune system through a small-molecule approach offers several unique advantages that are complementary to, and potentially synergistic with, biologic or cellular approaches. Such molecules have been shown to directly inhibit the signaling initiated by the respective ligands binding to their receptors, to recruit antibodies and other immunomodulatory molecules, or to promote or inhibit the proliferation of different immune cells to target specific types of cancer cells. Combined treatment of tumor patients by administration of such molecules together with MSC transfer might lead to surprising effects through the synergy of these two treatment agents.

In another preferred embodiment of the present invention the anti-tumor immunotherapy for combined administration comprises the administration of chimeric antigen receptor (CAR) T cells, wherein said T cell receptor binds specifically to a tumor-associated antigen, and the mesenchymal stem cells are not genetically modified.

### DETAILED DESCRIPTION OF THE INVENTION

The main response of the immune system towards tumors is to destroy the abnormal cells using killer T cells, sometimes with the assistance of helper T cells. However, most tumors release products that inhibit the immune response by various means and create an environment which generally counteracts T cell activation. The present invention provides means for supporting an anti-tumor immune reaction in such an environment by using the MSCs as described herein.

The invention is characterised by the surprising finding that MSCs show stimulatory adjuvant properties with respect to a combined anti-tumor immunotherapy. In particular, the MSCs of the invention - without transgenes expressing immune stimulating cytokines or other immune stimulatory molecules, such as those that inducing an antigen-independent immune response - lead to enhanced efficacy of CAR-T cells directed towards cancerous material.

The response to pathogens or cancerous tissue is orchestrated by complex interactions and activities of a large number of diverse cell types involved in an immune response. Immune cells as used herein may relate to any of the following cell types, as described in the context of the following processes: The innate immune response is the first line of defense and occurs soon after pathogen exposure or exposure to "foreign" or cancerous matter. It is carried out by phagocytic cells such as neutrophils and macrophages, cytotoxic natural killer (NK) cells, and granulocytes. The subsequent adaptive immune response includes antigen-specific defense mechanisms and may take days to develop. Cell types with critical roles in adaptive immunity are antigen-presenting cells including macrophages and dendritic cells. Antigen-dependent stimulation of various cell types, including T cell subsets and B cells, plays a critical role in host defense.

### Immune cells:

Immune cells as described herein relate to biological cells involved in the immune response in a subject. Immune cells are preferably selected from T Cells, B Cells, Dendritic Cells, Granulocytes, Innate Lymphoid Cells (ILCs), Megakaryocytes, Monocytes/Macrophages, Natural Killer (NK) Cells, Platelets, Red Blood Cells(RBCs) and/or Thymocytes.

T cells or T lymphocytes are a type of lymphocyte (a subtype of white blood cell) that play a central role in cell-mediated immunity. They can be distinguished from other lymphocytes, such as B cells and natural killer cells, by the presence of a T-cell receptor on the cell surface. The several subsets of T cells each have a distinct function. T cell subtypes include, without limitation, T helper type 1 (Th1) cells, T helper type 2 (Th2) cells, T helper type 9 (Th9) cells, T helper type 17 (Th17) cells, T helper type 22 (Th22) cells, Follicular helper T (Tfh) cells, Regulatory T (Treg) cells, Natural killer T (NKT) cells, Gamma delta T cells, CD8+ cytotoxic T lymphocytes (CTLs). Further non-limiting embodiments of T cells include thymocytes, immature T lymphocytes, mature T lymphocytes, resting T lymphocytes, cytokine-induced killer cells (CIK cells) or activated T lymphocytes. Cytokine-induced killer (CIK) cells are typically CD3- and CD56-positive, non-major histocompatibility complex (MHC)-restricted, natural killer (NK)-like T lymphocytes. The T cell can be a CD4+ T cell, a cytotoxic T cell (CTL; CD8+ T cell), CD4+CD8+ T cell, CD4 CD8 T cell, or any other subset of T cells.

### Chimeric antigen receptors (CARs) and CAR-T cells:

A T cell can be engineered with defined specificity by artificial T cell receptors (also known as chimeric T cell receptors, chimeric immunoreceptors, chimeric antigen receptors (CARs)) which are composite molecules and graft an arbitrary specificity onto an immune effector cell and activate the engineered cell upon engagement of cognate target. Typically, these receptors are used to graft the specificity of a monoclonal antibody onto a T cell linked to an intracellular signaling domain for activation.

Genetically engineered T cells may be created by infecting patient's cells with a retro- or lentivirus encoding a T cell receptor (TCR) or a CAR that is specific to recognize tumor antigens. Once the T cell binds to its target antigen, the stimulatory molecules provide the necessary signals for the T cell to become fully active. In this fully active state, the T cells can more effectively proliferate and can attack cancer cells.

For example, when reintroduced back to patients after autologous cell transplantation, the T cells modified with the CAR of the invention as described herein may recognize and kill tumor cells. CIK cells may have enhanced cytotoxic activity compared to other T cells, and therefore represent a preferred embodiment of an immune cell of the present invention. As would be understood by the skilled person, other cells may also be used as immune effector cells with the CARs as described herein. In particular, immune effector cells also include NK cells, NKT cells, neutrophils, and macrophages. Immune effector cells also include progenitors of effector cells wherein such progenitor cells can be induced to differentiate into an immune effector cells in vivo or in vitro.

The combined administration of the MSCs, with or without genetic modification, with immune cells, preferably T cells with or without genetic modification, such as those described herein, leads to a synergistic effect with respect to anti-cancer activity.

CARs are typically composed of an extracellular ectodomain derived from an antibody and an endodomain comprising signaling modules derived from T cell signaling proteins. In a preferred embodiment, the ectodomain preferably comprises variable regions from the heavy and light chains of an immunoglobulin configured as a single-chain variable fragment (scFv). The scFv is preferably attached to a hinge region that provides flexibility and transduces signals through an anchoring transmembrane moiety to an intracellular signaling domain. The transmembrane domains originate preferably from either CD8α or CD28. In the first generation of CARs the signaling domain consists of the zeta chain of the TCR complex. The term "generation" refers to the structure of the intracellular signaling domains. Second generation CARs are equipped with a single costimulatory domain originated from CD28 or 4-1BB. Third generation CARs already include two costimulatory domains, e.g. CD28, 4-1BB, ICOS or OX40, CD3 zeta.

In various embodiments, genetically engineered receptors that redirect cytotoxicity of immune effector cells toward cancer cells are provided. These genetically engineered receptors referred to herein as chimeric antigen receptors (CARs). CARs are molecules that combine antibody-based specificity for a desired antigen (e.g., a tumor-associated antigen) with a T cell receptor-activating intracellular domain to generate a chimeric protein that exhibits a specific anti-BCMA cellular immune activity. As used herein, the term, "chimeric," describes being composed of parts of different proteins or DNAs from different origins.

CARs contemplated herein, comprise an extracellular domain (also referred to as a binding domain or antigen-binding domain) that binds to a tumor-associated antigen, a transmembrane domain, and an intracellular domain, or intracellular signaling domain. Engagement of the anti-BCMA antigen binding domain of the CAR with BCMA on the surface of a target cell results in clustering of the CAR and delivers an activation stimulus to the CAR-containing cell. The main characteristic of CARs are their ability to redirect immune effector cell specificity, thereby triggering proliferation, cytokine production, phagocytosis or production of molecules that can mediate cell death of the target antigen expressing cell in a major histocompatibility (MHC) independent manner, exploiting the cell specific targeting abilities of monoclonal antibodies, soluble ligands or cell specific co-receptors.

In various embodiments, a CAR comprises an extracellular binding domain that comprises a humanized tumor-antigen-specific binding domain; a transmembrane domain; one or more intracellular signaling domains. In particular embodiments, a CAR comprises an extracellular binding domain that comprises a humanized anti-tumor-antigen antigen binding fragment thereof; one or more spacer domains; a transmembrane domain; one or more intracellular signaling domains.

The "extracellular antigen-binding domain" or "extracellular binding domain" provide a CAR with the ability to specifically bind to the target antigen of interest. The binding domain may be derived either from a natural, synthetic, semi-synthetic, or recombinant source. Preferred are scFV domains.

"Specific binding" is to be understood as via one skilled in the art, whereby the skilled person is clearly aware of various experimental procedures that can be used to test binding and binding specificity. Methods for determining equilibrium association or equilibrium dissociation constants are known in the art. Some cross-reaction or background binding may be inevitable in many protein-protein interactions; this is not to detract from the "specificity" of the binding between CAR and epitope. "Specific binding" describes binding of an anti-tumor-antigen antibody or antigen binding fragment thereof (or a CAR comprising the same) to a tumor antigen at greater binding affinity than background binding. The term "directed against" is also applicable when considering the term "specificity" in understanding the interaction between antibody and epitope.

An "antigen (Ag)" refers to a compound, composition, or substance that can stimulate the production of antibodies or a T cell response in an animal. In particular embodiments, the target antigen is a tumor-associated antigen, in other words an epitope of a polypeptide expressed predominantly in tumor cells. An "epitope" refers to the region of an antigen to which a binding agent binds. Epitopes can be formed both from contiguous amino acids or noncontiguous amino acids juxtaposed by tertiary folding of a protein.

"Single-chain Fv" or "scFv" antibody fragments comprise the VH and VL domains of antibody, wherein these domains are present in a single polypeptide chain and in either orientation {e.g., VL-VH or VH-VL). Generally, the scFv polypeptide further comprises a polypeptide linker between the VH and VL domains which enables the scFv to form the desired structure for antigen binding. In preferred embodiments, a CAR contemplated herein comprises antigen-specific binding domain that is an scFv and may be a murine, human or humanized scFv. Single chain antibodies may be cloned form the V region genes of a hybridoma specific for a desired target. In particular embodiments, the antigen-specific binding domain that is a humanized scFv that binds a human tumor-antigen polypeptide.

### Immune cells for cellular therapy

Dendritic cells (DCs) are antigen-presenting cells of the mammalian immune system. Their main function is to process antigen material and present it on the cell surface to the T cells of the immune system. They act as messengers between the innate and the adaptive immune systems. Dendritic cells are present in those tissues that are in contact with the external environment, such as the skin (where there is a specialized dendritic cell type called the Langerhans cell) and the inner lining of the nose, lungs, stomach and intestines. They can also be found in an immature state in the blood. Once activated, they migrate to the lymph nodes where they interact with T cells and B cells to initiate and shape the adaptive immune response. Owing to their properties, dendritic cells have become the natural agents for antigen delivery and vaccination strategies involving DCs have been developed. The aim of DC vaccination is to induce tumor-specific effector T cells that can reduce the tumor mass specifically and that can induce immunological memory to control tumor relapse. In this process, the first step is to provide DCs with tumor-specific antigens. This can be achieved by culturing ex vivo DCs that have been derived from patients with an adjuvant (that induces DC maturation) and the tumor-specific antigen, and then injecting these cells back into the patient, or by inducing DCs to take up the tumor-specific antigen in vivo. To improve the therapeutic use of DC vaccination strategies the co-administration of DCs together with MSC according the present invention is envisioned.

Monocytes are a type of white blood cells (leukocytes). They are the largest of all leukocytes. They are part of the innate immune system of vertebrates including all mammals (humans included). Monocytes are produced by the bone marrow from precursors called monoblasts, bipotent cells that differentiated from hematopoietic stem cells. Monocytes circulate in the bloodstream for about one to three days and then typically move into tissues throughout the body. They constitute between three to eight percent of the leukocytes in the blood. In tissues monocytes mature into different types of macrophages at different anatomical locations.

Macrophages, sometimes called macrophagocytes, are cells produced by the differentiation of monocytes in tissues. Monocytes and macrophages are phagocytes. Macrophages function in both non-specific defense (innate immunity) as well as help initiate specific defense mechanisms (adaptive immunity) of vertebrate animals. Macrophages predominantly expressing the killer phenotype are called M1 macrophages, whereas those involved in tissue repair are called M2 macrophages. The role of macrophages is to phagocytose, or engulf and then digest, cellular debris and pathogens, either as stationary or as mobile cells. They also stimulate lymphocytes and other immune cells to respond to pathogens (Palucka K et al. Nat Rev Cancer. 2012 Mar 22;12(4):265-77. doi: 10.1038/nrc3258). They are specialized phagocytic cells that attack foreign substances, infectious microbes and cancer cells through destruction and ingestion.

Innate lymphoid cells (ILCs) are a group of innate immune cells that belong to the lymphoid lineage (lymphocytes) but do not respond in an antigen-specific manner, as they lack a B or T cell receptor. This relatively newly described group of cells has different physiological functions, some of them analogous to helper T cells, while also including the cytotoxic NK cells. In accordance, they have an important role in protective immunity and the regulation of homeostasis and inflammation. Natural killer (NK) cells are cytotoxic innate effector cells analogous to the cytotoxic T cells of the adaptive immune system. They are distributed throughout the blood, organs, and lymphoid tissue and make up around 15% of the peripheral blood lymphocytes. NK cells play a role in tumor surveillance and the rapid elimination of virus-infected cells. They do not require the missing "self' signal of MHC Class I and can recognize stressed cells in the absence of antibodies, allowing them to react much more quickly than the adaptive immune system. Natural killer (NK) cells play critical roles in host immunity against cancer. In response, cancers develop mechanisms to escape NK cell attack or induce defective NK cells (Cheng M et al. Cell Mol Immunol. 2013 May;10(3):230-52. doi: 10.1038/cmi.2013.10. Epub 2013 Apr 22.). Current NK cell-based cancer immunotherapy aims to overcome NK cell paralysis using several approaches and the combined administration of MSCs might help to make NK cell based therapies more effective.

Granulocytes are a category of white blood cells characterized by the presence of granules in their cytoplasm encompassing neutrophils, eosinophils, basophils and mast cells.

### Immunotherapy

As used herein, "immunotherapy" comprises any kind of therapeutic approach or treatment directed against a tumor employing means of the immune system to negate or destroy tumor material. This includes, without limitation, immune checkpoint modulators, immune cell therapy, adoptive transfer of immune cells or other cells that modulate the immune response, modulation of the immune cells by small molecules or biopharmaceuticals such as monoclonal antibodies, cytokines, chemokines, and cancer treatment vaccines. Therefore, immunotherapy is to be understood in the context of the present invention to encompass a therapeutic agent that uses the immune system to treat cancer.

Immunotherapy encompasses, without limitation, cellular and biopharmaceutical and antibody therapy as well as the use of small molecules inducing a modification of the immune response to tumors.

Cellular therapies typically involve the administration of immune cells isolated from the blood or from a tumor of the patient. Immune cells directed towards the tumor to be treated are activated, cultured and returned to the patient where the immune cells attack the cancer. Cell types that can be used in this way are, without limitation, natural killer cells, lymphokine-activated killer cells, cytotoxic T cells, monocytes, macrophages, granulocytes and dendritic cells. Dendritic cell therapy provokes anti-tumor responses by causing dendritic cells to present tumor antigens. Dendritic cells present antigens to lymphocytes, which activates them, priming them to kill other cells that present the antigen.

One approach to these previous attempts at immunotherapy involves engineering patients' own immune cells to recognize and attack their tumors. And although this approach, called adoptive cell transfer (ACT), has been restricted to clinical trials so far, treatments using these engineered immune cells have generated therapeutic responses in patients with advanced cancer.

The present invention therefore encompasses adoptive cell transfer in combination with administration of the MSCs described herein. It is encompassed within the invention that administration of MSCs prior or simultaneous to immune cells (e.g. CAR-Ts) will enhance the anti-tumor activity of CAR-Ts, TCR-transgeneic T-cells, in vitro expanded T Cells or other immune effector cells administered during adoptive cell transfer. The presence of the MSC will enhances the activation of T-cells only locally, preferably within or in proximity to the tumor, and subsequently lead to a memory effector cell phenotype, thereby prolonging the therapeutic effect of the treatment.

Adoptive cell transfer uses T cell-based cytotoxic responses to attack cancer cells. T cells that have a natural or genetically engineered reactivity to a patient's cancer are generated in vitro and then transferred back into the cancer patient. Autologous tumor-infiltrating lymphocytes have been used as an effective treatment for patients with metastatic melanoma. This can be achieved by taking T cells that are found with the tumor of the patient, which are trained to attack the malignant cells. These T cells may be referred to as tumor-infiltrating lymphocytes (TIL). Such T cells may also be stimulated by the presence of MSC in vivo.

The present invention provides novel means for local and tissue-localized stimulation of an anti-tumor immune response, mediated preferably by TILs, engineered or natural cytotoxic T cells and/or NK cells.

Biopharmaceuticals, also known as a biologic(al) medical products, biologicals,or biologics, are any pharmaceutical drug product manufactured in, extracted from, or semisynthesized from biological sources. They include without limitation vaccines, blood, or blood components, and recombinant therapeutic protein such as antibodies, cytokines, chemokines and fusionproteins. Biopharmaceuticals can be composed of sugars, proteins, or nucleic acids or complex combinations of these substances. They are isolated from natural sources, including humans, animals, or microorganisms.

Antibodies are proteins produced by the immune system that bind to a target antigen on the cell surface. Those that bind to cancer antigens may be used to treat cancer. The MSCs of the present invention are capable of supporting and/or enhancing the cell- or antibody-based immunotherapies through their unique properties derived from the MSCs inherent properties.

The term "small molecule" relates to a low molecular weight (<900 daltons) organic compound that may help regulate a biological process. Modulating the immune system through a small molecule approach offers several unique advantages that are complementary to, and potentially synergistic with, other immunotherapies. Small molecules can act as antagonists to surface enzyme-linked receptors and receptors that interact with the tumor microenvironment, or even inhibit metabolic enzymes. Such molecules have been shown to directly inhibit the signaling initiated by the respective ligands binding to their receptors, to recruit antibodies and other immunomodulatory molecules, or to promote or inhibit the proliferation of different immune cells to target specific types of cancer cells.

Such immune response modifiers include for example imiquimod, antibody-recruiting molecules that target prostate cancer, integrin receptor antagonists, indoleamine-2,3-dioxygenase inhibitors, emodin, RORyt antagonists, ephrin receptor antagonists, membrane-bound carbonic anhydrase IX (CAIX) inhibitors, and selected protein kinase inhibitors as described by lyver VV (Anticancer Agents Med Chem. 2015;15(4):433-52.).

### Treatment:

The subject or patient, such as the subject in need of treatment or prevention, may be an animal, a vertebrate animal, a mammal, a rodent (e.g. a guinea pig, a hamster, a rat, a mouse), a murine (e.g. a mouse), a canine (e.g. a dog), a feline (e.g. a cat), an equine (e.g. a horse), a primate, a simian (e.g. a monkey or ape), a monkey (e.g. a marmoset, a baboon), an ape (e. g. gorilla, chimpanzee, orangutan, gibbon), or a human. The meaning of the terms "animal", "mammal", etc. is well known in the art and can, for example, be deduced from Wehner und Gehring (1995; Thieme Verlag). In the context of this invention, it is particularly envisaged that animals are to be treated which are economically, agronomically or scientifically important. Preferably, the subject/patient is a mammal. More preferably, the subject/patient is a human.

Cancer comprises a group of diseases that can affect any part of the body and is caused by abnormal cell growth and proliferation. These proliferating cells have the potential to invade the surrounding tissue and/or to spread to other parts of the body where they form metastasis. Worldwide, there were 14 million new cases of cancer and 8.2 million cancer related deaths in 2012 (World Cancer Report 2014). The majority of cancers is caused by environmental signals involving tobacco use, obesity and infections among others, while around 5-10% are genetic cases. Cancers can be classified into subcategories based on the cell of origin. The most common subcategories are carcinomas from epithelial cells, sarcomas from connective tissue and lymphomas and leukemias from hematopoietic cells. Cancer is associated with a high variety of local and systemic symptoms and cannot be cured in many cases. In light of the high number of new cancer patients and cancer related deaths novel treatment strategies are required.

Cancer according to the present invention refers to all types of cancer or neoplasm or malignant tumors found in mammals, including leukemias, sarcomas, melanomas and carcinomas. Either solid tumors and/or liquid tumors (such as leukemia or lymphoma) may be treated.

Leukemias include, but are not limited to acute nonlymphocytic leukemia, chronic lymphocytic leukemia, acute granulocytic leukemia, chronic granulocytic leukemia, acute promyelocytic leukemia, adult T-cell leukemia, aleukemic leukemia, a leukocythemic leukemia, basophylic leukemia, blast cell leukemia, bovine leukemia, chronic myelocytic leukemia, leukemia cutis, embryonal leukemia, eosinophilic leukemia, Gross' leukemia, hairy-cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocytic leukemia, micromyeloblastic leukemia, monocytic leukemia, myeloblastic leukemia, myelocytic leukemia, myeloid granulocytic leukemia, myelomonocytic leukemia, Naegeli leukemia, plasma cell leukemia, plasmacytic leukemia, promyelocytic leukemia, Rieder cell leukemia, Schilling's leukemia, stem cell leukemia, subleukemic leukemia, and undifferentiated cell leukemia.

Sarcomas include, but are not limited to a chondrosarcoma, fibrosarcoma, lymphosarcoma, melanosarcoma, myxosarcoma, osteosarcoma, Abernethy's sarcoma, adipose sarcoma, liposarcoma, alveolar soft part sarcoma, ameloblastic sarcoma, botryoid sarcoma, chloroma sarcoma, chorio carcinoma, embryonal sarcoma, Wilms' tumor sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, idiopathic multiple pigmented hemorrhagic sarcoma, immunoblastic sarcoma of B cells, lymphoma, immunoblastic sarcoma of T-cells, Jensen's sarcoma, Kaposi's sarcoma, Kupffer cell sarcoma, angiosarcoma, leukosarcoma, malignant mesenchymoma sarcoma, parosteal sarcoma, reticulocytic sarcoma, Rous sarcoma, serocystic sarcoma, synovial sarcoma, and telangiectaltic sarcoma.

Melanomas include, but are not limited to include, for example, acral-lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma, subungal melanoma, and superficial spreading melanoma.

Carcinomas include, but are not limited to acinar carcinoma, acinous carcinoma, adenocystic carcinoma, adenoid cystic carcinoma, carcinoma adenomatosum, carcinoma of adrenal cortex, alveolar carcinoma, alveolar cell carcinoma, basal cell carcinoma, carcinoma basocellulare, basaloid carcinoma, basosquamous cell carcinoma, bronchioalveolar carcinoma, bronchiolar carcinoma, bronchogenic carcinoma, cerebriform carcinoma, cholangiocellular carcinoma, chorionic carcinoma, colloid carcinoma, comedo carcinoma, corpus carcinoma, cribriform carcinoma, carcinoma en cuirasse, carcinoma cutaneum, cylindrical carcinoma, cylindrical cell carcinoma, duct carcinoma, carcinoma durum, embryonal carcinoma, encephaloid carcinoma, epiermoid carcinoma, carcinoma epitheliale adenoides, exophytic carcinoma, carcinoma exulcere, carcinoma fibrosum, gelatiniform carcinoma, gelatinous carcinoma, giant cell carcinoma, carcinoma gigantocellulare, glandular carcinoma, granulosa cell carcinoma, hair-matrix carcinoma, hematoid carcinoma, hepatocellular carcinoma, Hurthle cell carcinoma, hyaline carcinoma, hypernephroid carcinoma, infantile embryonal carcinoma, carcinoma in situ, intraepidermal carcinoma, intraepithelial carcinoma, Krompecher's carcinoma, Kulchitzky-cell carcinoma, large-cell carcinoma, lenticular carcinoma, carcinoma lenticulare, lipomatous carcinoma, lymphoepithelial carcinoma, carcinoma medullare, medullary carcinoma, melanotic carcinoma, carcinoma molle, mucinous carcinoma, carcinoma muciparum, carcinoma mucocellulare, mucoepidermoid carcinoma, carcinoma mucosum, mucous carcinoma, carcinoma myxomatodes, nasopharyngeal carcinoma, oat cell carcinoma, carcinoma ossificans, osteoid carcinoma, papillary carcinoma, periportal carcinoma, preinvasive carcinoma, prickle cell carcinoma, pultaceous carcinoma, renal cell carcinoma of kidney, reserve cell carcinoma, carcinoma sarcomatodes, schneiderian carcinoma, scirrhous carcinoma, carcinoma scroti, signet-ring cell carcinoma, carcinoma simplex, small-cell carcinoma, solanoid carcinoma, spheroidal cell carcinoma, spindle cell carcinoma, carcinoma spongiosum, squamous carcinoma, squamous cell carcinoma, string carcinoma, carcinoma telangiectaticurn, carcinoma telangiectodes, transitional cell carcinoma, carcinoma tuberosum, tuberous carcinoma, verrucous carcinoma, and carcinoma villosum.

Additional cancers include, but are not limited to Hodgkin's Disease, Non-Hodgkin's Lymphoma, multiple myeloma, neuroblastoma, breast cancer, ovarian cancer, lung cancer, rhabdomyosarcoma, primary thrombocytosis, primary macroglobulinemia, small-cell lung tumors, primary brain tumors, stomach cancer, colon cancer, malignant pancreatic insulanoma, malignant carcinoid, urinary bladder cancer, premalignant skin lesions, testicular cancer, lymphomas, thyroid cancer, esophageal cancer, genitourinary tract cancer, malignant hypercalcemia, cervical cancer, endometrial cancer, adrenal cortical cancer, and prostate cancer.

In some embodiments, "tumor" shall include, without limitation, a prostate tumor, a pancreatic tumor, a squamous cell carcinoma, a breast tumor, a melanoma, a basal cell carcinoma, a hepatocellular carcinoma, a choloangiocellular carcinoma, testicular cancer, a neuroblastoma, a glioma or a malignant astrocytic tumor such as glioblastma multiforme, a colorectal tumor, an endometrial carcinoma, a lung carcinoma, an ovarian tumor, a cervical tumor, an osteosarcoma, a rhabdo/leiomyosarcoma, a synovial sarcoma, an angiosarcoma, an Ewing sarcoma/PNET and a malignant lymphoma. These include primary tumors as well as metastatic tumors (both vascularized and non-vascularized).

### Mesenchymal stem cells:

The "mesenchymal stem cells" disclosed herein are preferably cells of non-haematopoietic origin that reside in the bone marrow and other tissues. The identification and nomenclature of the MSC has been clarified by the Mesenchymal and Tissue Stem Cell Committee of the International Society for Cellular Therapy (ISCT), who have proposed a set of minimal criteria to define MSC. Typically, MSCs can give rise to connective tissue, bone, cartilage, and cells in the circulatory and lymphatic systems. Mesenchymal stem cells are found in the mesenchyme, the part of the embryonic mesoderm that consists of loosely packed, fusiform or stellate unspecialized cells. As used herein, mesenchymal stem cells include, without limitation, CD34-negative stem cells.

In one embodiment of the invention, the mesenchymal stem cells are plastic-adherent cells, defined in some embodiments as multipotent mesenchymal stromal cells and also include CD34-negative cells. For the avoidance of any doubt, the term mesenchymal stem cell encompasses multipotent mesenchymal stromal cells that also includes a subpopulation of mesenchymal cells, MSCs and their precursors, which subpopulation is made up of multipotent or pluripotent self-renewing cells capable of differentiation into multiple cell types in vivo.

As used herein, CD34-ngeative cell shall mean a cell lacking CD34, or expressing only negligible levels of CD34, on its surface. CD34-negative cells, and methods for isolating such cells, are described, for example, in Lange C. et al., "Accelerated and safe expansion of human mesenchymal stromal cells in animal serum-free medium for transplantation and regenerative medicine". J. Cell Physiol. 2007, Apr. 25.

Mesenchymal stem cells can be distinguished from hematopoietic stem cells (HSCs) by a number of indicators. For example, HSCs are known to float in culture and to not adhere to plastic surfaces. In contrast, mesenchymal stem cells adhere to plastic surfaces. The CD34-negative mesenchymal stem cells of the present invention are adherent in culture.

### Genetic modification

MSCs or any other therapeutic cell, such as the immune cells described herein, may be genetically modified to express an exogenous nucleic acid, such as a transgene, for example a therapeutic gene product from an exogenous nucleic acid. As used herein, "genetically modified" shall mean any kind of cell or organism carrying modified genetic material such as nucleic acids. As used herein "nucleic acid" shall mean any nucleic acid molecule, including, without limitation, DNA, RNA and hybrids or modified variants thereof. An "exogenous nucleic acid" or "exogenous genetic element" relates to any nucleic acid introduced into the cell, which is not a component of the cells "original" or "natural" genome. Exogenous nucleic acids may be integrated or non-integrated in the genetic material of the target mesenchymal stem cell, or relate to stably transduced nucleic acids.

Genetic modification of a mammalian cell can be determined by skilled person using commonly available techniques. For example, sequencing of the genome or parts thereof of a modified cell is possible, thereby identifying if exogenous nucleic acids are present. Alternatively, other molecular biological techniques may be applied, such as the polymerase chain reaction (PCR), to identify/amplify exogenous genetic material. Exogenous nucleic acids may be detected by vector sequences, or parts of vector sequences remaining at the site of genetic modification. In cases where vector sequences (for example vector sequences flanking a therapeutic transgene) can be removed from the genome, the addition of a therapeutic transgene may still be detected by sequencing efforts by detecting genomic sequences incorporating a therapeutic gene at a "non-natural" position in the genome.

Any given gene delivery method is encompassed by the invention and preferably relates to viral or non-viral vectors, as well as biological or chemical methods of transfection. The methods can yield either stable or transient gene expression in the system used.

Genetically modified viruses have been widely applied for the delivery of genes into stem cells. A viral vector may be employed in the genetic modification of the present invention.

Preferred viral vectors for genetic modification of the MSCs described herein relate to retroviral vectors, in particular to gamma retroviral vectors. The gamma retrovirus (sometimes referred to as mammalian type C retroviruses) is a sister genus to the lentivirus clade, and is a member of the Orthoretrovirinae subfamily of the retrovirus family. The Murine leukemia virus (MLV or MuLV), the Feline leukemia virus (FeLV), the Xenotropic murine leukemia virus-related virus (XMRV) and the Gibbon ape leukemia virus (GALV) are members of the gamma retrovirus genus. A skilled person is aware of the techniques required for utilization of gamma retroviruses in genetic modification of MSCs. For example, the vectors described Maetzig et al (Gammaretroviral vectors: biology, technology and application, 2001, Viruses Jun;3(6):677-713) or similar vectors may be employed. For example, the Murine Leukemia Virus (MLV), a simple gammaretrovirus, can be converted into an efficient vehicle of genetic therapeutics in the context of creating gamma retrovirus-modified MSCs and expression of a therapeutic transgene from said MSCs after delivery to a subject.

Adenoviruses may be applied, or RNA viruses such as Lentiviruses, or other retroviruses. Adenoviruses have been used to generate a series of vectors for gene transfer cellular engineering. The initial generation of adenovirus vectors were produced by deleting the EI gene (required for viral replication) generating a vector with a 4kb cloning capacity. An additional deletion of E3 (responsible for host immune response) allowed an 8kb cloning capacity. Further generations have been produced encompassing E2 and/or E4 deletions. Lentiviruses are members of Retroviridae family of viruses (M. Scherr et al., Gene transfer into hematopoietic stem cells using lentiviral vectors. Curr Gene Ther. 2002 Feb; 2(1):45-55). Lentivirus vectors are generated by deletion of the entire viral sequence with the exception of the LTRs and cis acting packaging signals. The resultant vectors have a cloning capacity of about 8 kb. One distinguishing feature of these vectors from retroviral vectors is their ability to transduce dividing and non-dividing cells as well as terminally differentiated cells.

Non-viral methods may also be employed, such as alternative strategies that include conventional plasmid transfer and the application of targeted gene integration through the use of integrase or transposase technologies. These represent approaches for vector transformation that have the advantage of being both efficient, and often site-specific in their integration. Physical methods to introduce vectors into cells are known to a skilled person. One example relates to electroporation, which relies on the use of brief, high voltage electric pulses which create transient pores in the membrane by overcoming its capacitance. One advantage of this method is that it can be utilized for both stable and transient gene expression in most cell types. Alternative methods relate to the use of liposomes or protein transduction domains. Appropriate methods are known to a skilled person and are not intended as limiting embodiments of the present invention.

The present invention therefore encompasses adoptive cell transfer in combination with administration of the MSCs described herein. It is encompassed within the invention that administration of MSCs prior or simultaneous to immune cells (e.g. CAR-Ts) will enhance the anti-tumor activity of CAR-Ts, TCR-transgeneic T-cells, in vitro expanded T Cells or other immune effector cells administered during adoptive cell transfer. The presence of the MSCs will enhances the activation of T-cells either systemically and/or locally, preferably within or in proximity to the tumor, but also may lead to activation or stimulation of the CAR-T cells prior to administration in a mixture of therapeutic cells.

### Administration

The present invention encompasses treatment of a patient by introducing a therapeutically effective number of cells into a subject's bloodstream. As used herein, "introducing" cells "into the subject's bloodstream" shall include, without limitation, introducing such cells into one of the subject's veins or arteries via injection. Such administering can also be performed, for example, once, a plurality of times, and/or over one or more extended periods. A single injection is preferred, but repeated injections over time (e.g., weekly, monthly, quarterly, half-yearly or yearly) may be necessary in some instances. Such administering is also preferably performed using an admixture of CD34-negative cells and a pharmaceutically acceptable carrier. Pharmaceutically acceptable carriers are well known to those skilled in the art and include, but are not limited to, 0.01-0.1 M and preferably 0.05 M phosphate buffer or 0.8% saline, as well as commonly used proprietary cryopreservation media.

Administration may also occur locally, for example by injection into an area of the subject's body in proximity to a tumor disease. MSCs have been shown to migrate towards cancerous tissue. Regardless, the local administration of the cells as described herein may lead to high levels of the cells at their site of action.

Additionally, such pharmaceutically acceptable carriers can be aqueous or non-aqueous solutions, suspensions, and emulsions, most preferably aqueous solutions. Aqueous carriers include water, alcoholic/aqueous solutions, emulsions and suspensions, including saline and buffered media. Parenteral vehicles include sodium chloride solution, Ringer's dextrose, dextrose and sodium chloride, lactated Ringer's and fixed oils. Intravenous vehicles include fluid and nutrient replenishers, electrolyte replenishers such as Ringer's dextrose, those based on Ringer's dextrose, and the like. Fluids used commonly for i.v. administration are found, for example, in Remington: The Science and Practice of Pharmacy, 20th Ed., p. 808, Lippincott Williams S-Wilkins (2000). Preservatives and other additives may also be present, such as, for example, antimicrobials, antioxidants, chelating agents, inert gases, and the like.

In one embodiment, a therapeutically effective number of cells is administered. This may relate to either the MSCs of the invention or a therapeutic immune cell as the combined anti-tumor immune therapy. As used herein, a "therapeutically effective number of cells" includes, without limitation, the following amounts and ranges of amounts: (i) from about 1 x 10² to about 1 x 10⁸ cells/kg body weight; (ii) from about 1 x 10³ to about 1 x 10⁷ cells/kg body weight; (iii) from about 1 x 10⁴ to about 1 x 10⁶ cells/kg body weight; (iv) from about 1 x 10⁴ to about 1 x 10⁵ cells/kg body weight; (v) from about 1 x 10⁵ to about 1 x 10⁶ cells/kg body weight; (vi) from about 5 x 10⁴ to about 0.5 x 10⁵ cells/kg body weight; (vii) about 1 x 10³ cells/kg body weight; (viii) about 1 x 10⁴ cells/kg body weight; (ix) about 5 x 10⁴ cells/kg body weight; (x) about 1 x 10⁵ cells/kg body weight; (xi) about 5 x 10⁵ cells/kg body weight; (xii) about 1 x 10⁶ cells/kg body weight; and (xiii) about 1 x 10⁷ cells/kg body weight. Human body weights envisioned include, without limitation, about 5 kg, 10 kg, 15 kg, 30 kg, 50 kg, about 60 kg; about 70 kg; about 80 kg, about 90 kg; about 100 kg, about 120 kg and about 150 kg. These numbers are based on pre-clinical animal experiments and human trials and standard protocols from the transplantation of CD34+ hematopoietic stem cells. Mononuclear cells (including CD34+ cells) usually contain between 1:23000 to 1:300000 CD34-negative cells. These cell numbers may be applied for MSCs or for the anti-tumor cellular immunotherapy.

As used herein, "treating" a subject afflicted with a disorder shall mean slowing, stopping or reversing the disorder's progression. In the preferred embodiment, treating a subject afflicted with a disorder means reversing the disorder's progression, ideally to the point of eliminating the disorder itself. As used herein, ameliorating a disorder and treating a disorder are equivalent. The treatment of the present invention may also, or alternatively, relate to a prophylactic administration of said cells. Such a prophylactic administration may relate to the prevention of any given medical disorder, or the prevention of development of said disorder, whereby prevention or prophylaxis is not to be construed narrowly under all conditions as absolute prevention. Prevention or prophylaxis may also relate to a reduction of the risk of a subject developing any given medical condition, preferably in a subject at risk of said condition.

The subject from which the mesenchymal stem cells and/or immune cells are obtained may be the same subject, to whom the cells are intended to be administered after cultivation. Such cells may therefore be considered autologous. The cells may however be obtained from a subject distinct from the intended patient, therefore being considered allogenic. As used herein, a cell is "allogenic" with respect to a subject if it or any of its precursor cells are from another subject of the same species. As used herein, a cell is "autologous" with respect to a subject if it or its precursor cells are from the same subject. In a preferred embodiment, the immune cells are autologous to the subject of medical treatment.

The cell(s) described herein may comprise different types of carriers depending on whether it is to be administered in solid, liquid or aerosol form, and whether it need to be sterile for such routes of administration as injection. The present invention can be administered intravenously, intradermally, intraarterially, intraperitoneally, intralesionally, intracranially, intraarticularly, intraprostaticaly, intrapleurally, intratracheally, intranasally, intravitreally, intravaginally, intrarectally, topically, intratumorally, intramuscularly, intraperitoneally, subcutaneously, subconjunctival, intravesicularlly, mucosally, intrapericardially, intraumbilically, intraocularally, orally, topically, locally, inhalation (e.g., aerosol inhalation), injection, infusion, continuous infusion, localized perfusion bathing target cells directly, via a catheter, via a lavage, in cremes, in lipid compositions (e.g., liposomes), or by other method or any combination of the forgoing as would be known to one of ordinary skill in the art (see, for example, Remington's Pharmaceutical Sciences, 18th Ed. Mack Printing Company, 1990, incorporated herein by reference).

### Combined administration:

"Combined administration" may relate to concurrent and/or sequential administration of said mesenchymal stem cells prior to, during and/or subsequent to said immunotherapy (preferably CAR-T cell treatment). Combined treatment shall also include a combination treatment regime comprising multiple administrations of either therapeutic component of the treatment. Further embodiments of combined administration are provided herein.

Combined administration encompasses simultaneous treatment, co-treatment or joint treatment, and includes the administration of separate formulations of MSCs with immunotherapies, such as checkpoint inhibitors and/or immune cells, whereby treatment may occur within minutes of each other, in the same hour, on the same day, in the same week or in the same month as one another. Sequential administration of any given combination of combined agents (for example MSCs, immune cells and/or checkpoint inhibitors) is also encompassed by the term "combined administration". A combination medicament, comprising one or more of said MSCs with another immunotherapeutic, such as checkpoint inhibitors and/or immune cells, may also be used in order to co-administer the various components in a single administration or dosage.

A combined immunotherapy may precede or follow treatment with MSCs, which are preferably not genetically modified, or have genetic modifications but without exogenous nucleic acids comprising regions encoding immune-stimulatory cytokines, by intervals ranging from minutes to weeks. In embodiments where the other immunotherapeutic agent and the MSCs are administered separately to the site of interest, one would generally ensure that a significant period of time did not expire between the time of each delivery, such that the agent and the MSCs would still be able to exert an advantageously combined effect on a treatment site. In such instances, it is contemplated that one would contact the cell with both modalities within about 12-24 h of each other and, more preferably, within about 6-12 h of each other, with a delay time of only about 12 h being preferred. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations.

### Immune stimulatory molecules and cytokines:

In one embodiment the invention relates to mesenchymal stem cells (MSCs) for use in the treatment of a tumor and/or as an adjuvant for an anti-tumor immunotherapy, wherein said treatment comprises the combined administration of said mesenchymal stem cells with an anti-tumor immunotherapy and wherein said MSCs are either not genetically modified, or are genetically modified, but do not comprise exogenous nucleic acids that encode immune-stimulatory cytokines or immune stimulatory molecules that induce an antigen-independent immune response.

The term "immune-stimulatory molecule" refers to any molecule that induces an activation or stimulation of the immune system or any component or part of the immune system. This activation can be antigen-dependent or antigen-independent.

As used herein, "antigen-dependent" or "antigen-specific" activation of the immune system relates to the activation of T- and B-cells by antigen recognition through the TCR and BCR, respectively, leading to the activation of the adaptive immune-system.

As used herein, "antigen-independent" activation of the immune system relates to any kind of stimulation of the immune system that is not dependent on antigen-specific activation of T- and B-cells. This may relate to, but is not limited to, attracting immune effector cells, promoting the maturation of memory immune cells, inducing inflammation or other immune response that is not antigen-specific as mentioned herein. These immune responses (or aspects of immune responses) occur regardless of the particular antigen against which the immune response may (subsequently) react. As such, this kind of immune system activation can be referred to as "antigen-independent". Cytokines are examples of known antigen-independent stimulators or modulators of the immune system in the meaning of the present invention.

In the case of antigen-independent immune stimulation, the immune system may subsequently or in combination carry out one or more antigen-dependent (-specific) reactions against particular antigens. For example, an antigen-independent immune stimulation leads to activation of the immune system in general (e. g. by enriching effector cells), which then is primed to carry out any given antigen-specific immune response via activated T- and B-cells (e. g. a subsequent or concordant antigen-specific immune reaction by an activated T cell against a tumor-specific antigen). The "stimulatory molecules that induce an antigen-independent immune response" may therefore be understood as molecules (such as cytokines) that comprise a stimulatory function for an antigen-independent immune response, but in some embodiments may or may not also comprise a stimulatory function for an antigen-dependent immune response.

Immune-stimulatory cytokines are one embodiment of molecules that induce an antigen-independent immune stimulation. In this context, immune-response stimulatory cytokines may be able to attract immune effector cells, such as T cells, and promote the maturation of memory immune cells. Alternatively, the antigen-independent immune stimulation may lead to inflammatory conditions at the site of stimulation. Examples of these cytokines, without limitation, are IFN gamma, IL-2, IL-12, IL-23, IL-15 and IL-21.

In one embodiment, the MSCs administered in combination with the anti-tumor immune therapy are genetically modified and comprise a transgene expressing a cytotoxic or other therapeutic protein. An immune response may occur that is directed against such a cytotoxic or therapeutic protein product. For the purposes of the invention, this immune reaction is considered an antigen-dependent immune reaction. In some embodiments such genetically modified MSCs expressing such cytotoxic or therapeutic proteins (that do not induce an antigen-independent immune response) are not excluded from the scope of the invention.

In some embodiments of the invention the immune-stimulatory molecules that induce an antigen-independent immune response, or the immune stimulatory cytokines
In a preferred embodiment the invention relates to mesenchymal stem cells (MSCs) for use in the treatment of a tumor, wherein said treatment comprises the combined administration of said mesenchymal stem cells with an anti-tumor immunotherapy and wherein said MSCs are genetically modified, but do not comprise exogenous nucleic acids that encode immune response-stimulating cytokines.

In some embodiments the immune response stimulating cytokines are those molecules described in WO 2016/026854, which is (including any US counterparts) hereby incorporated by reference.

As known in the art, an immune response-stimulating cytokine is to be understood as any cytokine that leads to or produces either directly or indirectly the induction, activation and/or enhancement of an immune response, preferably directed against an antigen, for example a tumor antigen. In particular, the immune response-stimulating cytokines of the invention are preferably considered as cytokines that leads to the induction, activation and/or enhancement of an immune response beneficial for the treatment of a tumor disease.

The present invention is in some embodiments characterised in that immune response-stimulating cytokines are expressed from endogenous genes, but not from exogenous genes.

The disclaimer disclosed herein regarding the absence of an exogenous nucleic acid encoding an immune response-modulating cytokine, immune response-associated cytokine or immune-response stimulating cytokine may be applied to any one or more of the cytokines disclosed herein. The terms "stimulation" and "activation" of the "immune system" or of an "immune response" may be used interchangeably.

### Cytokines:

Cytokines are a diverse group of non-antibody proteins that act as mediators between cells. Cytokines are currently being clinically used as biological response modifiers for the treatment of various disorders. The term cytokine is a general term used to describe a large group of proteins. Particular kinds of cytokines may include Monokines, namely cytokines produced by mononuclear phagocytic cells, Lymphokines, namely cytokines produced by activated lymphocytes, especially Th cells, Interleukins, namely cytokines that act as mediators between leukocytes and Chemokines, namely small cytokines primarily responsible for leucocyte migration. Cytokine signaling is flexible and can induce both protective and damaging responses. They can produce cascades, or enhance or suppress production of other cytokines. Despite the various roles of cytokines, a skilled person is aware of which cytokines may be considered as immune response-stimulating and therefore applied in the treatment of a tumor disease as described herein.

The following cytokines may be referred to as immune-response stimulatory or immune response-modulatory cytokines. Two commonly used groups of cytokines in anti-tumor therapy are the interferons and interleukins.

Interferons are cytokines produced by the immune system usually involved in an anti-viral response, but also show effectiveness in the treatment of cancer. There are three groups of interferons (IFNs): type I (IFN alpha and IFN beta), type 2 (IFN gamma) and the relatively newly discovered type III (IFN lambda). IFN alpha has been applied in the treatment of hairy-cell leukaemia, AIDS-related Kaposi's sarcoma, follicular lymphoma, chronic myeloid leukaemia and melanoma. Type I and II IFNs have been researched extensively and although both types promote the anti-tumor effects of the immune system, only type I IFNs have been shown to be clinically effective in cancer treatment so far. IFN lambda has been tested for its anti-tumor effects in animal models, and shows promise.

According to some embodiments of the present invention, the immune-response stimulatory or immune response-modulatory cytokines (terms may be used interchangeably) are preferably those involved in T cell regulation or with effector function for T cells (T cell regulatory cytokines). These cytokines exhibit desired properties with respect to inducing a pro-inflammatory microenvironment and thereby facilitating the activation of the immune system against the tumor and/or enhance the efficacy of anti-tumor immunotherapeutic treatments. Such cytokines may be able to attract immune effector cells, such as T cells, and promote the maturation of memory immune cells. Examples of these cytokines are IFN gamma, IL-2, IL-12, IL-23, IL-15 and IL-21 (refer Kelley's Textbook of Rheumatology; Firestein et al, 8th ed. (ISBN 978-1-4160-3285-4), p367 "Cytokines").

The immune response-stimulating cytokine may be an inflammatory chemokine. In one embodiment the immune response-stimulating cytokine is a chemokine with chemotactic properties for attracting T cells, for example, CCL1, CCL2 and/or CCL17. Chemokines refer to a sub-group of cytokines (signalling proteins) secreted by cells. Cytokines have the ability to induce directed chemotaxis in nearby responsive cells; they are chemotactic cytokines. Proteins are classified as chemokines according to shared structural characteristics such as small size (typically approximately 8-10 kilodaltons in size), and the presence of four cysteine residues in conserved locations that are key to forming their 3-dimensional shape. Cytokines may be known under alternative definitions, such as the SIS family of cytokines, SIG family of cytokines, SCY family of cytokines, Platelet factor-4 superfamily or intercrines. Chemokines have been classified into four main subfamilies: CXC, CC, CX3C and XC. All of these proteins exert their biological effects by interacting with G protein-linked transmembrane receptors called chemokine receptors, which are selectively found on the surfaces of their target cells. Examples of inflammatory chemokines relate to CXCL-8, CCL2, CCL3, CCL4, CCL5, CCL11 and CXCL10, CXCL1, CXCL2.

### Further definitions:

The term "immunomodulatory functions" relates to functions or properties of molecules or cells that induce a changes or modulation in the function, action or status of any component of the immune system.

The term "stroma" as used herein refers to the supportive framework of a tissue or an organ (or gland, tissue or other structure), usually composed of extracellular matrix (ECM) and stromal cells. The stroma is distinct from the parenchyma, which consists of the key functional elements of that organ. Stromal cells (in the dermis layer) adjacent to the epidermis (the very top layer of the skin) release growth factors that promote cell division. Stroma is made up of the non-malignant host cells. Stroma provides an extracellular matrix on which tumors can grow or maintain existence or separate themselves from the immune environment.

As used herein, the term "tumor microenvironment" relates to the cellular environment in which any given tumor exists, including the tumor stroma, surrounding blood vessels, immune cells, fibroblasts, other cells, signalling molecules, and the ECM.

As used herein "cell migration" or "homing" is intended to mean movement of a cell towards a particular chemical or physical signal. Cells often migrate in response to specific external signals, including chemical signals and mechanical signals. The MSCs as described herein are capable of homing to tumor tissue or other inflammation signals.

Chemotaxis is one example of cell migration regarding response to a chemical stimulus. In vitro chemotaxis assays such as Boyden chamber assays may be used to determine whether cell migration occurs in any given cell.

For example, the cells of interest may be purified and analysed. Chemotaxis assays (for example according to Falk et al., 1980 J. Immuno. Methods 33:239-247) can be performed using plates where a particular chemical signal is positioned with respect to the cells of interest and the transmigrated cells then collected and analysed. For example, Boyden chamber assays entail the use of chambers isolated by filters, used as tools for accurate determination of chemotactic behaviour. The pioneer type of these chambers was constructed by Boyden (Boyden (1962) "The chemotactic effect of mixtures of antibody and antigen on polymorphonuclear leucocytes". J Exp Med 115 (3): 453). The motile cells are placed into the upper chamber, while fluid containing the test substance is filled into the lower one. The size of the motile cells to be investigated determines the pore size of the filter; it is essential to choose a diameter which allows active transmigration. For modelling in vivo conditions, several protocols prefer coverage of filter with molecules of extracellular matrix (collagen, elastin etc.) Efficiency of the measurements can be increased by development of multiwell chambers (e.g. NeuroProbe), where 24, 96, 384 samples are evaluated in parallel. Advantage of this variant is that several parallels are assayed in identical conditions.

As used herein "engraftment" relates to the process of incorporation of grafted or transplanted tissue or cells into the body of the host. Engraftment may also relate to the integration of transplanted cells into host tissue upon administration and their survival and under some conditions differentiation into non-stem cell states.

Techniques for assessing engraftment, and thereby to some extent both migration and the biodistribution of MSCs, can encompass either in vivo or ex vivo methods. Examples of in vivo methods include bioluminescence, whereby cells are transduced to express luciferase and can then be imaged through their metabolism of luciferin resulting in light emission; fluorescence, whereby cells are either loaded with a fluorescent dye or transduced to express a fluorescent reporter which can then be imaged; radionuclide labelling, where cells are loaded with radionuclides and localized with scintigraphy, positron emission tomography (PET) or single photon emission computed tomography (SPECT); and magnetic resonance imaging (MRI), wherein cells loaded with paramagnetic compounds (e.g., iron oxide nanoparticles) are traced with an MRI scanner. Ex vivo methods to assess biodistribution include quantitative PCR, flow cytometry, and histological methods. Histological methods include tracking fluorescently labelled cells; in situ hybridization, for example, for Y-chromosomes and for human-specific ALU sequences; and histochemical staining for species-specific or genetically introduced proteins such as bacterial β-galactosidase. These immunohistochemical methods are useful for discerning engraftment location but necessitate the excision of tissue. For further review of these methods and their application see Kean et al., MSCs: Delivery Routes and Engraftment, Cell-Targeting Strategies, and Immune Modulation, Stem Cells International, Volume 2013 (2013).

As used herein, in "proximity with" a tissue includes, for example, within 50 mm, 10 mm, 5 mm, within 1 mm of the tissue, within 0.5 mm of the tissue and within 0.25 mm of the tissue.

The specific molecules mentioned herein relate preferably to mammalian molecules, preferably human molecules. For example, the cytokines and/or chemokines mentioned herein relate preferably to the human sequences, as can be obtained by a skilled person without undue effort, for example from a sequence database such as those maintained by the National Center for Biotechnology Information (NCBI). Protein sequences or protein-coding nucleic acid sequences may be modified in comparison to commonly known sequences, for example by making e. g. conservative amino acid substitutions in a protein sequence, or by using the degeneracy of the genetic code in order to change the coding sequence without changing the encoded protein sequence. As a skilled person is aware, the sequences of biological molecules can be changed (mutated) via standard techniques, their properties thereby being improved or maintained in comparison to the known original sequence. Any modified cytokine sequence that maintains the basic properties of, or is functionally analogous to, the known sequence is therefore encompassed by the present invention.

### FIGURES

The following figures are presented in order to describe particular embodiments of the invention, by demonstrating a practical implementation of the invention, without being limiting to the scope of the invention or the concepts described herein.

### Short description of the figures:

Fig. 1: #946 CAR expression on CEA-specific human CAR-T cells by two colour FACS.
Fig. 2: Wild-type MSC enhance the anti-tumor activity of CAR-T cell.
Fig. 3: Mesenchymal stem cells do not suppress CAR-T cell activation.
Fig. 4: MSC support proliferation and survival of CAR-T cells in activation dependent manner.
Fig. 5: Survival of activated CAR T cells in presence of MSC.
Fig. 6: Native MSC increase NK-cell dependent killing of tumor cells.
Fig. 7: Native MSC support CD4+ T cell proliferation and activation in vitro.
Fig. 8: Genetically engineered MSC which express the transgene alpha-1 anti-trypsin activate T cell proliferation in a comparable manner as native MSC.

### Detailed description of the figures:

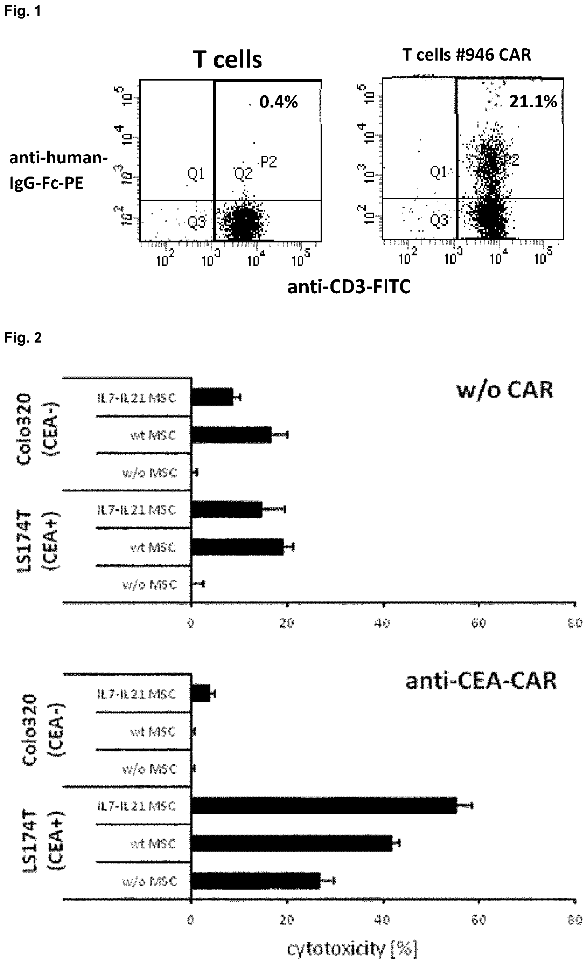

Fig. 1: #946 CAR expression on CEA-specific human CAR-T cells by two colour FACS.

Human peripheral T cells were isolated from the blood of a healthy donor and transduced by vector #946 encoding the sequence of a CAR consisting of an anti-Carcinoembryonic antigen (CEA) single chain Fv-binding domain, a human IgG1 derived hinge-CH2-CH3 spacer domain and a chimeric transmembrane and intracellular CD28-CD3zeta domain for combined CD28-CD3zeta signaling with specificity for carcinoembryonic antigen (CEA). 12 hrs upon end of transduction cells were tested for #946 CAR expression by two colour FACS using a PE-labeled polyclonal goat-anti-human IgG Fc antibody and the FITC-labeled murine anti-CD3 human monoclonal antibody UCHT1.

Fig. 2: Wild-type MSC enhance the anti-tumor activity of CAR-T cell

CEA is highly expressed on the surface of a majority of pancreatic adenocarcinomas. CEA expression is elevated in nearly 90% of cancers arising from endodermal tissue, including the gastrointestinal tract, pulmonary tissues, and breast. Anti-CEA-CAR grafted T cells and non-modified T cells w/o CAR for control (each 2x10(4)/well) were co-cultivated for 48h with CEA+ LS174T or CEA- Colo320 tumor cells (each 2.5x10(4)/well) and wt or IL7/IL21 secreting MSC (each 3x10(3)/well) in 96 well round bottom plates. Viability of tumor cells was determined by a tetrazolium salt based XTT-assay. Cytolysis [%] was determined by 100-viability [%]. Values represent mean of triplicates +/- standard deviation (SD).

Fig. 3: Mesenchymal stem cells do not suppress CAR-T cell activation

Anti-CEA-CAR grafted T cells and non-modified T cells w/o CAR for control (each 2x10(4)/well) were co-cultivated for 48h with CEA+ LS174T or CEA- Colo320 tumor cells (each 2.5x10(4)/well) and wt or IL7/IL21 secreting MSC (each 3x10(3)/well) in 96 well round bottom plates. Supernatants were harvested and IFN-gamma content was determined by ELISA. Values represent mean of experimental triplicates +/- standard deviation.

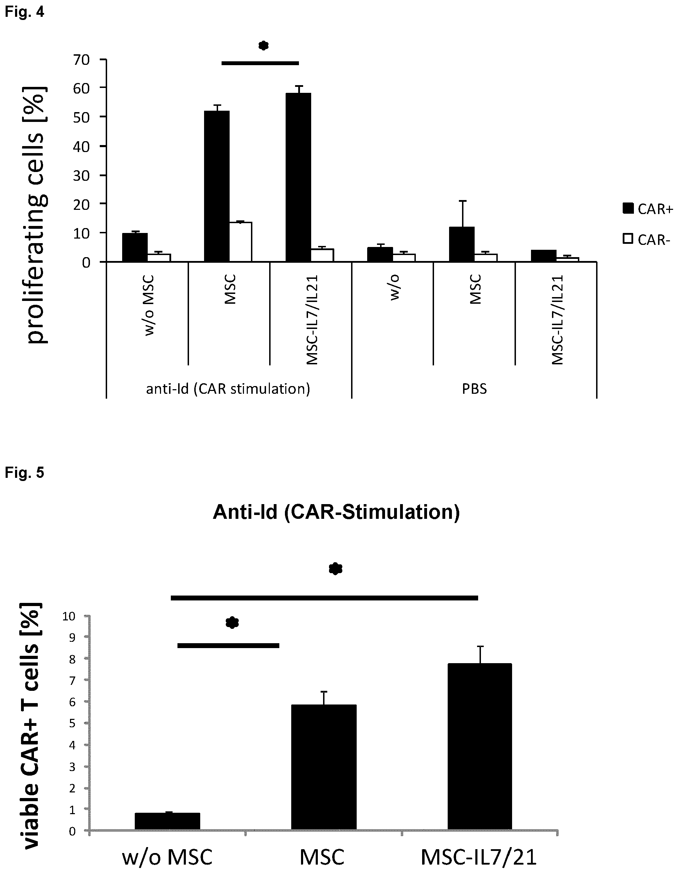

Fig. 4: MSC support proliferation and survival of CAR-T cells in activation dependent manner.

Modulation of CAR T cell proliferation upon CAR-stimulation by solid phase bound anti-idiotypic antibody in presence of MSC. Anti-CEA-CAR T cells (#946-CAR) were CFSE labeled and co-cultivated (5x10(4)/well) with non-modified or IL7/IL21 secreting MSC (5000/well), respectively, in 96-well plates that were coated with solid phase bound anti-idiotypic mAb BW2064 (4 µg/ml) or PBS for control. After 5 days cells were recovered, CAR T cells stained with an anti-human IgG-PE antibody and analyzed by flow cytometry. CFSE dilution and the number of proliferating cells were determined. Values represent mean of triplicates +/- standard deviation (SD). Significant differences were determined by Student's T test.

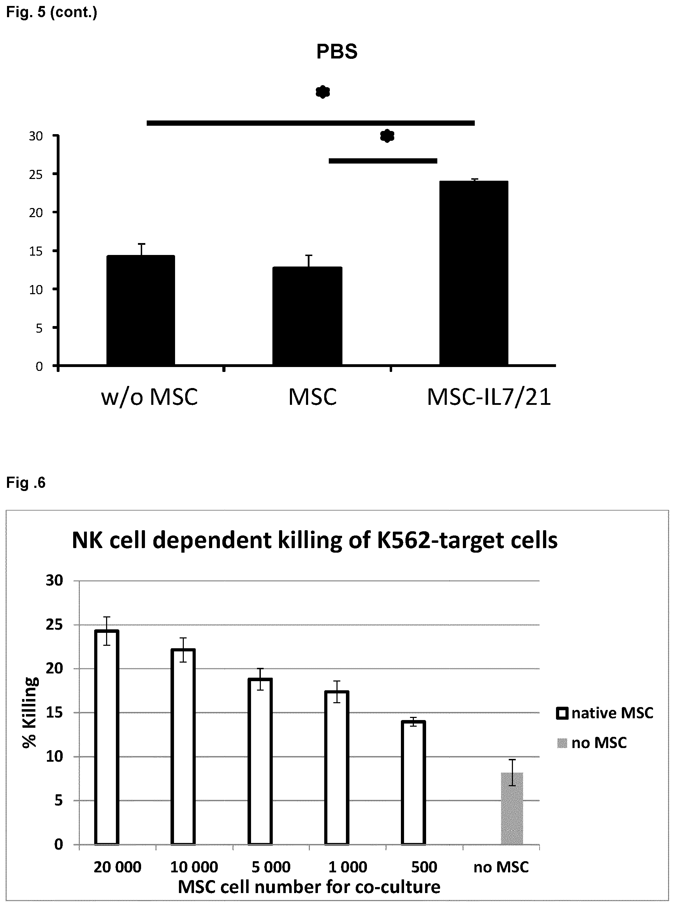

Fig. 5: Survival of activated CAR T cells in presence of MSC.

Anti-CEA-CAR T cells (#946-CAR) were CFSE labeled and co-cultivated (5x10(4)/well) with non-modified or IL7/IL21 secreting MSC (5000/well), respectively, in 96-well plates that were coated with solid phase bound anti-idiotypic mAb BW2064 (4 µg/ml) or PBS for control. After 6 days cells were recovered, stained with an anti-human IgG-PE antibody to identify CAR T cells and 7-AAD for discrimination of life and dead cells. The cells were analyzed by flow cytometry. The percentage of living CAR-positive T cells relative to all T-cells was determined. Values represent mean of triplicates +/- standard deviation (SD).

Fig. 6: Native MSC increase NK-cell dependent killing of tumor cells.

Human native MSC were seeded in different concentrations (20 000/10 000/5000/1000/500 cells per 12-well) and incubated overnight in RPMI medium with 10% Fetal Calf Serum and 1% Glutamin. Human NK cells were isolated by negative MACS sorting frozen PBMC's using the "NK Cell isolation kit" (Miltenyi, 130-092-657) according to the instructions of the manufacturer. The NK cell purity is assessed by FACS measurement using a CD56 specific antibody staining (Miltenyi, 130-100-683) according to the instructions of the manufacturer.

The purified NK cells (3x10^5 per sample) are co-cultured overnight with the plated native MSC or alone. On the next day an NK cytotoxicity assay is performed. The target tumor cell line K562 (1x10^6 cells) was stained with 10µM Calcein and incubated for 30min at 37°C (1mM, C3099, Lot 1687887, Life Technologies). Afterwards the target cell line was incubated with the NK cells with an effector-target (E:T). 5000 NK and 5000 target cells were seeded into a well of 96 well-plate. The plate was incubated at 37°C in 5% CO2 for 4 h. Supernatant was collected and the release of calcein as read-out for killing was measured in Fluorometer (excitation filter: 485 ± 9 nm; bandpass filter: 530 ± 9 nm). Killing was normalized relative to calcein-stained K562 that were lysed with triton X (Sigma Aldrich). Incubation of NK cells with MSC increases their killing activity in a dose dependent manner.

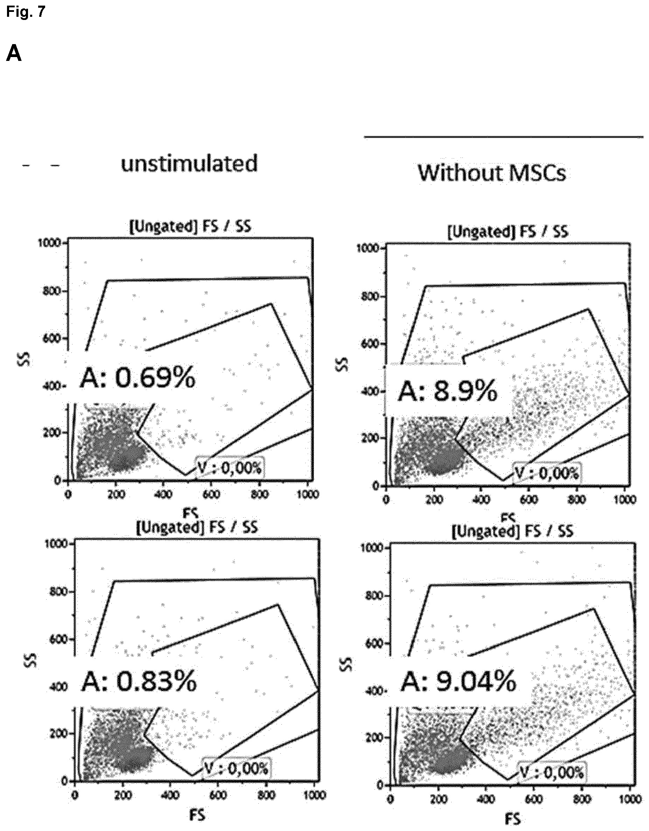

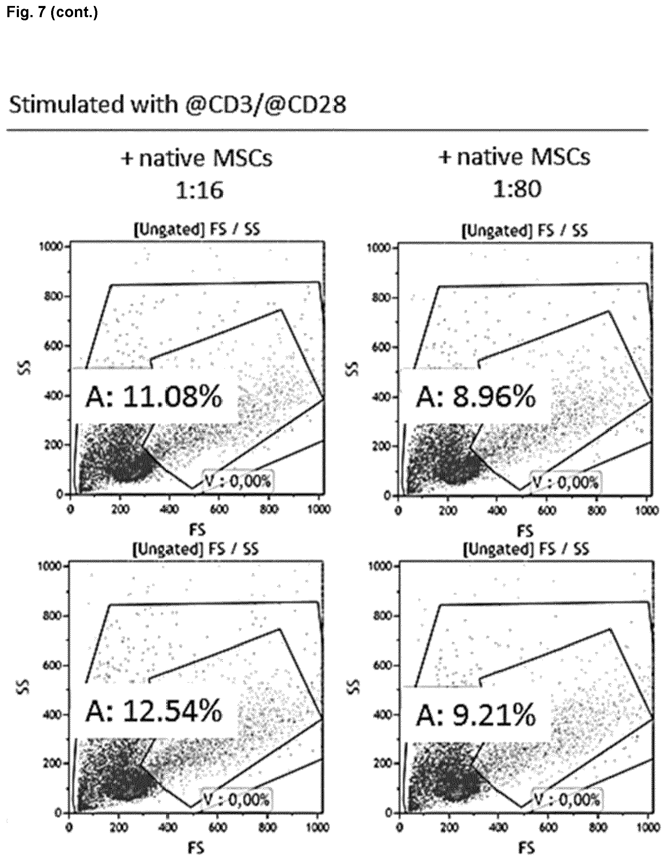

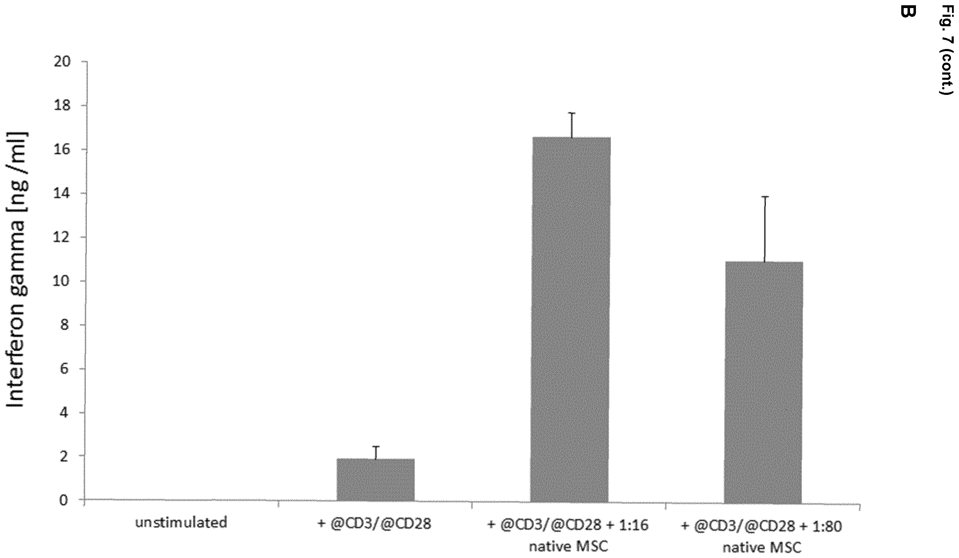

Fig. 7: Native MSC support CD4+ T cell proliferation and activation in vitro.

Human CD4+ cells were isolated from frozen PBMC using the "CD4 T cell isolation kit" (Miltenyi, 130-096-533) according to the instructions of the manufacturer. The CD4 cells were seeded into a 24-well plate (4x10^5 per well in 600µl) in RPMI1640 medium + 10% FCS + 1% Glutamax. CD4 cells were stimulated with anti-CD3 and anti-CD28 antibodies (Becton-Dickinson, 1µg/ml aCd3 5µg/ml aCd28). MSC were seeded in the well at a ratio (MSC:PBMC) of 1:16 or 1:80. The cultures were incubated for 3 days at 37°C. Supernatants were collected to detect IFNg, Proliferation was detected by FACS-analysis. A) The percentage of activated and proliferating CD4 cells was increased in the presence of MSC as seen in the FCS/SSC FACS analysis. Duplicates are displayed. B) The release of IFNg as sign of CD4-T cell activation was increased by the presence of MSC in a dose dependent manner.

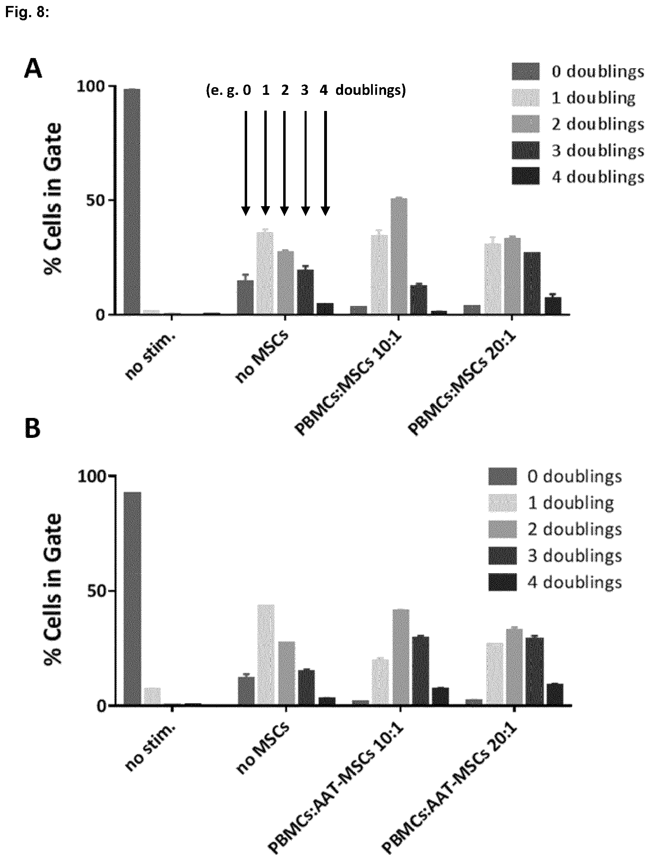

Fig. 8: Genetically engineered MSC which express the transgene alpha-1 anti-trypsin activate T cell proliferation in a comparable manner as native MSC.

4x10^5 PBMC per well (stained with 5mM CFSE, molecular probes, CellTrace Cell Proliferation Kits, C34554, according to the instructions of the manufacturer) were cocultured with MSC in the indicated ratios in a 24-well plate (200µl RPMI-1640 medium + 10%FCS and 1% Glutamax). PMBC were stimulated with stimulated with anti-CD3 and anti-CD28 antibodies (Becton-Dickinson, 1µg/ml aCd3 5µg/ml aCd28). Cells were incubated for 3 days at 37°C. Proliferation of lymphocytes was analysed by FACS. Calcein signal intensity was used to determine the number of doublings that had occurred. The analysis indicated the coculture of PBMC with (A) native and (B) genetically modified MSC increased the proliferation of PBMC. In each case a larger fraction of the lymphocytes had undergone 1 or more doublings compared to the sample without any MSCs. Furthermore, the percentage of PBMC that had not undergone any doublings was reduced in the presence of MSC.

### EXAMPLES

The following examples are presented in order to describe particular and in some cases preferred embodiments of the invention, by demonstrating a practical implementation of the invention, without being limiting to the scope of the invention or the concepts described herein.

### Preparation of CEA-specific human CAR-T cells

The #946 Chimeric antigen receptor consists of an anti-Carcinoembryonic antigen (CEA) single chain Fv-binding domain, a human IgG1 derived hinge-CH2-CH3 spacer domain and a chimeric transmembrane and intracellular CD28-CD3zeta domain for combined CD28-CD3zeta signaling. The CD28 signaling domain was furthermore mutated to abrogate IL2 secretion. The gamma-retroviral vector coding for the #946 CAR was produced according to SOP-VectProd using a Galv envelope. In summary vector particle production was done transiently on the human embryonic kidney cell line 293T after lipofectamin (R) mediated DNA transfection. Vector particles were pseudotyped with Galv. No vector titer was determined. Supernatants were filtered through 0.45 µm membrane and loaded on poly-D-lysin (PDL) coated culture plates by centrifugation for 30 min at 1500xg.

Transduction of human blood lymphocytes was carried out accordingly. Human T cells were isolated from the blood of a healthy donor and separated by Ficoll density centrifugation. Mononuclear cells were seeded at a density of 5x10⁶ cells/ml in RPMI 1640 + 10% FCS (v/v) and activated by 100 ng/ml anti-CD3 mAb OKT3 and 400 U/ml IL2. After 96 hrs cells were harvested, resuspended in Xvivo15 medium + 200 U/ml IL2 at a density of 5 x10⁶ cells/ml and transduced on virus loaded plates by centrifugation for 90 min at 1500 x g in fresh supernatant of transfected 293T cells. The cells were incubated for 12 hrs. Cells were recovered and tested for #946 immunoreceptor expression (Fig. 1).

### Preparation of human mesenchymal stem cells

The MSC were prepared according to the apceth process, for example as described using the cell culture medium disclosed in US8557578 B2, which is hereby incorporated in its entirety by reference. The cells were isolated from bone marrow from healthy human donors by plastic adherence and are cultured in growth medium.

Human MSCs are isolated from bone marrow by plastic adherence and are cultured in growth medium e.g. FBS containing DMEM as described by Pittinger, M.F. (2008) Mesenchymal stem cells from adult bone marrow, In D.J. Prockop, D.G. Phinney, B.A. Bunnell, Methods in Molecular Biology 449, Mesenchymal stem cells, Totowa: Humana Press), or in the culture medium as described in US8557578 B2.

### Generation of vectors for the expression of cytokines and chemokine:

The transgene expression cassettes consisting a promoter and a gene (e.g. cDNA) for an immunostimulatory factor or factor supporting immune response are constructed using standard cloning techniques as described in Julia Lodge, Peter Lund, Steve Minchin (2007) Gene Cloning, New York: Tylor and Francis Group. The promoters may be constitutive promoters like the CMV promoter or the PGK promoter or inducible promoters like Tie2, RANTES or the HSP70 promoter.

Examples for genes encoding immunostimulatory factors or factors supporting immune responses are IL-2, IL-7, IL-15, IL-21, IL-12, IFN gamma, IFN beta, SDF-1, CCL23, CCL19, CCL1, CCL2, CCL17, CCL22 and/or CCL4 (Strengell et al., M, The Journal of Immunology,2003, 170: 5464 -5469; Borish et al., J Allergy Clin Immunol. 2003 Feb;111(2 Suppl): S460-7). The gene may or may not be fused with tag-sequences (e.g. marker proteins / peptides like the hemagglutinin-tag or the HIS-tag) to allow easy detection of expression later on (Hinrik Garoff, 1985, Annual Review of Cell Biology, Vol. 1: 403-445).

The transgene is then inserted into a suitable vector system (e.g. lentiviral or gamma-retroviral vector) by standard cloning techniques. A suitable vector is for example described by Baum (EP 1757703 A2). The vector may or may not include a second transgene cassette consisting of a promoter and a selectable marker gene (cell surface marker or resistance gene, for example the pac gene to confer puromycin resistance) to allow enrichment of genetically modified cells later in the process (David P. Clark, Nanette J. Pazdernik, 2009, Biotechnology: Applying the Genetic Revolution, London: Elsevier).

### Genetic modification of mesenchymal stem cell:

The transduction is performed with modifications as described by Murray et al., 1999 Human Gene Therapy. 10(11): 1743-1752 and Davis et al., 2004 Biophysical Journal Volume 86 1234-1242. In detail:
6-well cell culture plates (e.g. Corning) are coated with Poly-L-Lysine (PLL) (e.g. Sigma-Aldrich, P4707-50ML): The PLL solution (0.01%) is diluted to final concentration between 0.0001% and 0.001% with PBS. 2ml of the diluted PLL are used for each well. The plate is incubated at least for 2h at room temperature. After incubation, the plates are washed carefully with PBS.

Viral vector supernatant in a final volume of 2ml is added to each PLL-coated well. The number of particles should between 2x10e3 and 1x10e6 per well, which will result in multiplicity of infection of 0.25 and 10. The loaded plate is centrifuged for 2000x g, 30min, 4°C. Afterwards the supernatant is discarded and 1x10e5 mesenchymal stem cells are seeded per well. The plates are incubated at 37° with 5% CO2 for further use.

### Combinatorial treatment of a mouse tumor model with MSCs and antigen specific CAR-T cells demonstrates synergistic effect

Immune-competent mice that express the CEA transgene (CEAtg) in the intestinal and pulmonary tracts (Chmielewski et al.; Gastroenterology. 2012 Oct;143(4):1095-107.e2. doi: 10.1053/j.gastro.2012.06.037. Epub 2012 Jun 27.) are given intrapancreatic injections of Panc02 CEA(+) cells (express CEA and click beetle luciferase) and tumors are grown for 10 days. Mice are then given single intravenous injections of T cells engineered to express a chimeric antigen receptor (CAR) with specificity for CEA either alone, or together with wt MSCs (not genetically modified), or with MSCs overexpressing and secreting IL7 and IL21. WT MSCs or MSCs expressing IL7 and IL21 are also injected alone without co-injection of CAR T cells. Injection of the anti-CEA CAR T cells alone slightly reduces the size of pancreatic tumors, whereas injection of WT MSCs alone has no effect on the tumor. MSCs expressing IL7 and IL21 has only slight anti-tumor effects. However, combinatorial injection of anti-CEA CAR T cells with MSCs (WT or IL7/IL21 expressing MSCs) reduces tumor size below the limit of detection in all mice and produces long-term tumor eradication.

The examples are supplemented by the figures, in which the surprising effect of the MSCs described herein without genetic modification is demonstrated with respect to enhancing the anti-tumor effect of CAR-Ts.

### Combinatorial treatment of a mouse tumor model with MSCs and NK cells demonstrates synergistic effect

Immune-deficient mice are subjected to intracranial injection of Glioblastoma cell line U87 expressing luciferase and tumors are grown for 3 days. Mice are then subjected to single intravenous injections of isolated human NK-cells. In addition, mice receive intracranial injections with wt MSCs (not genetically modified), or with MSCs overexpressing and secreting IL7 and IL21. WT MSCs or MSCs expressing IL7 and IL21 are also injected alone, without co-injection of NK cells. Injection of the NK cells alone slightly reduces the size of pancreatic tumors, whereas injection of WT MSCs alone has no effect on the tumor. MSCs expressing IL7 and IL21 has only slight anti-tumor effects. However, combinatorial injection of NK cells with MSCs (WT or IL7/IL21 expressing MSCs) reduces tumor size below the limit of detection in all mice and produces long-term tumor eradication (Fig. 6).

### Combinatorial treatment of a mouse tumor model with MSCs and adoptive T cell transfer demonstrates synergistic effect

Immune-deficient mice are subjected to intrahepatic injections of HUH7 liver carcinoma cells expressing luciferase and tumors are grown for 14 days. Mice are then subjected to single intravenous injections of isolated human T cells or together with wt MSCs (not genetically modified), or with MSCs overexpressing and secreting IL7 and IL21. WT MSCs or MSCs expressing IL7 and IL21 are also injected alone without co-injection of T cells. Injection of the T cells alone slightly reduces the size of pancreatic tumors, whereas injection of WT MSCs alone has no effect on the tumor. MSCs expressing IL7 and IL21 has only slight anti-tumor effects. However, combinatorial injection of NK cells with MSCs (WT or IL7/IL21 expressing MSCs) reduces tumor size below the limit of detection in all mice and produces long-term tumor eradication.

### Combinatorial treatment of a mouse tumor model with MSCs and the check point inhibitor (anti-PD-L1 antibody) therapy demonstrates synergistic effect

The experiment can be performed as described by Deng et al. (Deng et al. (2014) Irradiation and anti-PD-L1 treatment synergistically promote antitumor immunity in mice, J Clin Invest. 2014;124(2):687-695) with modifications:
Six- to 8-week old BALB/c mice are purchased from Harlan. All mice are maintained under specific pathogen-free conditions. The cell line MC38 is a colon adenocarcinoma cell line. MC38 tumor cells (1 x 106) are injected s.c. into the flanks of mice. MC38 are allowed to grow for about 8 days. For the PD-L1 blockade experiment, 100 µg anti-PD-L1 antibody (clone 10F.9G2; Bio-XCell) is administered i.p. to mice every 3 days for a total of four times. In addition, mice receive MSC treatment alone (up to 3 injections with doses between 1x10^3 - 1x10^6 MSC per mouse) or in combination with anti-PD-L1. Anti-PD-L1 treatment leads to a decrease in tumor volume. Combination of anti-PD-L1 and MSC leads essentially to eradication of the tumor, while treatment with MSC alone only leads to slight decrease in tumor volume.

### Combinatorial treatment of a mouse tumor model with MSCs and IDO inhibitor therapy demonstrates synergistic effect

The experiment can be performed as described by Nakamura et al.( Nakamura et al.(2015) Effects of indoleamine 2,3-dioxygenase inhibitor in non-Hodgkin lymphoma model mice.Int J Hematol.102(3):327-34) with modifications:
Female BALB/c mice aged 6-8 weeks are obtained from Charles River. A20, a BALB/c B-cell lymphoma cell line originally derived from a spontaneous reticulum cell neoplasm can be obtained from American Type Culture Collection (Manassas, VA, USA). The cell line is cultured in complete RPMI 1640 medium with 10 % FBS, 2 mM 1-glu-tamine, 100 U/mL penicillin, 100 U/mL streptomycin, and 50µM 2-ME at 37 °C in a humidified 5 % CO2 incubator.

To establish tumors, 1 x 10e6 A20 cells are resuspended in 200 µL of PBS and s.c. injected into the lower back of naïve syngeneic BALB/c mice. Seven days later treatment is initiated. The IDO inhibitor 1-methyl-d-tryptophan (d-1MT) (Sigma-Aldrich, St. Louis, MO, USA) is dissolved in sterile water (5 mg/mL) and adjusted to pH 9.9 with NaOH. The mice were fed with d-1MT - supplemented water. In addition, mice received injections of MSCs alone (up to 3 injections with doses between 1x10^3 - 1x10^6 MSC per mouse) or in combination with d-1MT -supplemented water. Tumor growth is monitored two times per week by caliper measurement. The tumor volume are calculated. The mice are killed by cervical dislocation on day 28. Compared to untreated control mice d-1MT treatment results in reduced tumor growth. Treatment with MSC alone leads to slight tumor reduction. Combination of the IDO inhibitor with MSC leads essentially to tumor eradication in the mice.

### Native MSC support CD4+ T cell proliferation and activation in vitro

As shown in Figure 7, the percentage of activated and proliferating CD4 cells is increased in the presence of MSC after co-culturing in vitro, as seen in the FCS/SSC FACS analysis. Furthermore, the release of IFNg as sign of CD4-T cell activation was increased by the presence of MSC in a dose dependent manner, indicating the ability of MSCs to activate CD4 T cells.

### Genetically engineered MSC which express the transgene alpha-1 anti-trypsin (which do not expressing immune-stimulating cytokines from the exogenous nucleic acid) activate T cell proliferation in a comparable manner as native MSC

As shown in Figure 8, the co-culture of PBMC with either (A) native or (B) genetically modified MSC lead to an increase in the proliferation of PBMC. In each case a larger fraction of the lymphocytes had undergone 1 or more doublings compared to the sample without any MSCs. This provides support that genetically modified MSCs, without exogenous immune-stimulatory cytokines, are also capable of stimulating immune cell responses. Furthermore, the percentage of PBMC that had not undergone any doublings was reduced in the presence of MSC.

## Claims

1. A mesenchymal stem cell (MSC) for use in the treatment of a tumor, wherein said treatment comprises the combined administration of said MSC with an anti-tumor immunotherapy, and wherein said MSC does not comprise exogenous nucleic acids that encode immune response-stimulating cytokines.

2. The mesenchymal stem cell for use according to claim 1, wherein the MSCs are not genetically modified.

3. The mesenchymal stem cell for use according to claim 1, wherein the MSCs are genetically modified but do not comprise exogenous nucleic acids that encode immune response-stimulating cytokines.

4. The mesenchymal stem cell for use according to any of claims 1 to 3, wherein the anti-tumor immunotherapy for combined administration comprises the administration of an immune checkpoint modulator.

5. The mesenchymal stem cell for use according to the preceding claim, wherein the immune checkpoint modulator is a checkpoint inhibitor, such as antibodies against CTLA-4, PD-1, PD-L1.

6. The mesenchymal stem cell for use according to any of claims 1 to 3, wherein the anti-tumor immunotherapy for combined administration comprises the administration of an antibody that binds specifically to a tumor-associated antigen.

7. The mesenchymal stem cell for use according to any of claims 1 to 3, wherein the anti-tumor immunotherapy for combined administration comprises the administration of a cytokine or chemokine.

8. The mesenchymal stem cell for use according to any of claims 1 to 3, wherein the anti-tumor immunotherapy for combined administration comprises the administration of a small molecule with anti-tumor immune-stimulating properties.

9. The mesenchymal stem cell for use according to any of claims 1 to 3, wherein the anti-tumor immunotherapy for combined administration comprises the administration of immune cells.

10. The mesenchymal stem cell for use according to the preceding claim, wherein the immune cells for combined administration are T cells, preferably wherein the T cells for combined administration express a chimeric antigen receptor (CAR), wherein said CAR binds specifically to a tumor-associated antigen.

11. The mesenchymal stem cell for use according to claim 9, wherein the immune cells for combined administration are dendritic cells.

12. The mesenchymal stem cell for use according to claim 9, wherein the immune cells for combined administration are not dendritic cells.

13. The mesenchymal stem cell for use according to claim 9, wherein the immune cells for combined administration are macrophages and/or monocytes innate lymphoid cells, NK cells granulocytes.

14. The mesenchymal stem cell for use according to claim 1 or 9, wherein the mesenchymal stem cells and/or immune cells for combined administration are autologous to the subject of medical treatment.

15. The mesenchymal stem cell for use according to claim 1 or 9, wherein the mesenchymal stem cells and/or immune cells for combined administration are allogeneic to the subject of medical treatment.
